# EUROPÄISCHE PATENTSCHRIFT

(11) **EP 0 344 519 B1**
(45) Veröffentlichungstag und Bekanntmachung des Hinweises auf die Patenterteilung: **14.04.1993**
(21) Anmeldenummer: 89108895.7
(22) Anmeldetag: 18.05.1989
(51) Int. Cl.: C07D 215/14, C07D 215/18, A61K 31/47

(54) **Substituierte 4-(Chinolin-2-yl-methoxy)phenyl-essigsäure-Derivate**
Substituted 4-(quinolin-2-yl-methoxy)phenyl-acetic-acid derivatives
Acides (quinolyl-2-méthoxy)-4-phényl acétiques et leurs esters substitués

(30) Priorität: 31.05.1988 DE 3818443; 06.01.1989 DE 3900261
(43) Veröffentlichungstag der Anmeldung: 06.12.1989
(73) Patentinhaber: BAYER AG, 51368 Leverkusen (DE)
(72) Erfinder: Mohrs, Klaus, Dr., D-5600 Wuppertal 1 (DE); Raddatz, Siegfried, Dr., D-5000 Koeln 80 (DE); Perzborn, Elisabeth, Dr., D-5600 Wuppertal 11 (DE); Fruchtmann, Romanis, D-5000 Koeln 1 (DE); Kohlsdorfer, Christian, Dr., D-5042 Erftstadt (DE); Müller-Peddinghaus, Reiner, Prof. Dr., D-5060 Bergisch-Gladbach (DE); Theisen, Pia, D-5060 Bergisch-Gladbach 2 (DE)

(56) Entgegenhaltungen:
- EP-A- 0 181 568
- EP-A- 0 206 751

## Beschreibung

Die Erfindung betrifft substituierte 4-(Chinolin-2-yl-methoxy)phenylessigsäuren, deren Ester und Amide, Verfahren zu ihrer Herstellung und ihre Verwendung in Arzneimitteln.

Es ist bekannt, daß 3-(Chinolin-2-yl-methoxy)phenylessigsäure und 2-[3-(Chinolin-2-yl-methoxy)phenyl]propionsäure und deren Methyl- und Ethylester eine antiinflammatorische und antiallergische Wirkung besitzen [vgl. EP-A 181 568].

Es wurden substituierte 4-(Chinolin-2-yl-methoxy)-phenylessigsäuren, deren Ester und Amide der allgemeinen Formel (I) 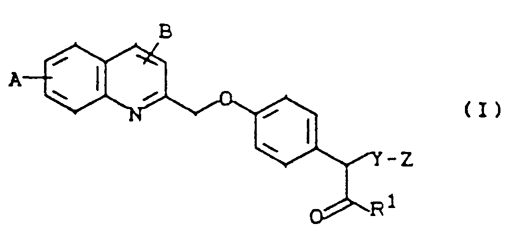
in welcher
- R¹: - für eine Gruppe der Formel
-OR² oder 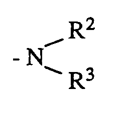 steht, wobei
R² und R³ gleich oder verschieden sind und
- für Wasserstoff, C₁-C₆-Alkyl Benzyl, Phenyl oder eine Gruppe der Formel 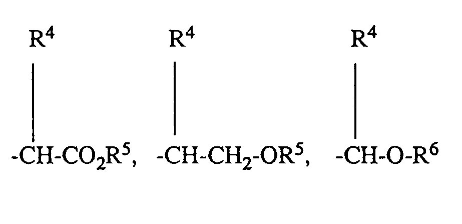 oder 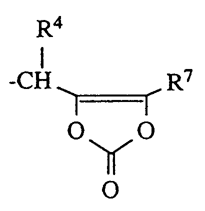 stehen, wobei
- R⁴: - für Wasserstoff, C₁-C₆-Alkyl, Phenyl oder Benzyl steht, das gegebenenfalls substituiert sein kann durch Hydroxy, C₁-C₆-Alkoxycarbonyl, Carboxyl, C₁-C₆-Alkylthio, Thienyl, Furyl, Pyridyl, Pyrimidyl, Pyrazinyl, Pyridazinyl, Chinolyl, Isochinolyl, Chinazolyl, Chinoxalyl, Thiazolyl, Benzothiazolyl, Isothiazolyl, Oxazolyl, Benzoxazolyl, Isoxazolyl, Imidazolyl, Benzimidazoly, Pyrazolyl, Indolyl und Isoindolyl oder Carbymoyl,
- R⁵: - für Wasserstoff, C₁-C₆-Alkyl, Phenyl oder Benzyl steht,
- R⁶: - für eine Gruppe der Formel -COR⁵ oder -CO₂R⁵ steht, wobei R⁵ die oben angegebene Bedeutung hat,
und
- R⁷: - für Wasserstoff, C₁-C₆-Alkyl oder Phenyl steht,
- Y: - für eine Gruppe der Formel 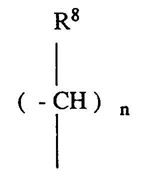 steht, wobei
- R⁸: - für Wasserstoff, C₁-C₆-Alkyl oder Phenyl steht,
und
- n: - eine Zahl von 0 bis 5 bedeutet,
- Z: - für Norbornyl steht, oder für eine Gruppe der Formel 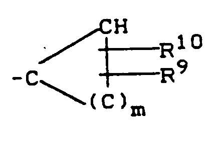 oder 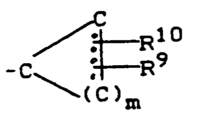 steht, wobei
- R⁹ und R¹⁰: gleich oder verschieden sind und Wasserstoff, C₁-C₆-Alkyl oder Phenyl bedeuten, oder
- R⁹ und R¹⁰: gemeinsam einen gesättigten carbocyclischen Ring mit bis zu 6 Kohlenstoffatomen bilden können und
- m: eine Zahl von 1 bis 6 bedeutet,
und
- A und B: gleich oder verschieden sind und Wasserstoff, Methyl, Ethyl, Fluor, Chlor oder Brom bedeuten,

sowie deren Salze
gefunden.

Im Vergleich zu den aus EP-A 181 568 bekannten Verbindungen haben die erfindungsgemäßen Chinoline der allgemeinen Formel (I) überraschenderweise eine höhere in vitro Aktivität als Leukotriensynthesehemmer und eine stärkere in vivo Wirkung nach oraler Applikation.

Im Rahmen der vorliegenden Erfindung werden physiologisch unbedenkliche Salze bevorzugt. Physiologisch unbedenkliche Salze der substituierten 4-(Chinolin-2-yl-methoxy)phenylessigsäuren, -ester und -amide können Salze der erfindungsgemäßen Stoffe mit Mineralsäuren, Carbonsäuren oder Sulfonsäuren sein. Besonders bevorzugt sind z.B. Salze mit Chlorwasserstoffsäure, Bromwasserstoffsäure, Schwefelsäure, Phosphorsäure, Methansulfonsäure, Ethansulfonsäure, Toluolsulfonsäure, Benzolsulfonsäure, Naphthalindisulfonsäure, Essigsäure, Propionsäure, Milchsäure, Weinsäure, Zitronensäure, Fumarsäure, Maleinsäure oder Benzoesäure.

Salze im Rahmen der vorliegenden Erfindung sind außerdem Salze der einwertigen Metalle wie Alkalimetalle und die Ammoniumsalze. Bevorzugt werden Natrium-, Kalium- und Ammoniumsalze.

Bevorzugte Verbindungen der allgemeinen Formel (I) sind solche, in denen
- R¹: - für eine Gruppe der Formel
-OR² oder 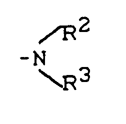 steht, wobei
- R² und R³: gleich oder verschieden sind und
- für Wasserstoff, Methyl, Ethyl, Propyl, Isopropyl, Butyl, tert.Butyl, Phenyl oder Benzyl stehen, oder für eine Gruppe der Formel 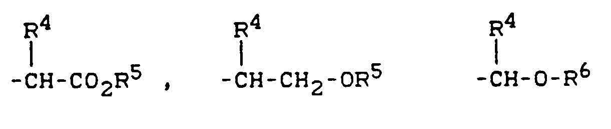 oder 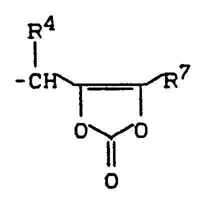 stehen, wobei
- R⁴: - für Wasserstoff, Methyl, Ethyl, Propyl, Isopropyl, Butyl, tert.Butyl, Benzyl oder Phenyl steht, das gegebenenfalls substituiert sein kann durch Hydroxy, Methoxycarbonyl, Ethoxycarbonyl, Propoxycarbonyl, Carboxyl, Methylthio, Ethylthio, Propylthio, Imidazolyl oder Carbamoyl,
- R⁵: - für Wasserstoff, Methyl, Ethyl, Propyl, Isopropyl, Butyl, tert.Butyl, Phenyl oder Benzyl steht,
- R⁶: - für eine Gruppe der Formel -COR⁵ oder -CO₂R⁵ steht, wobei
R⁵ die oben angegebene Bedeutung hat
und
- R⁷: - für Wasserstoff, Methyl, Ethyl, Propyl, Isopropyl, Butyl, tert. Butyl oder Phenyl steht,
- Y: - für eine Gruppe der Formel 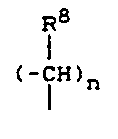 steht, wobei
- R⁸: - für Wasserstoff, Methyl, Ethyl, Propyl, Isopropyl, Butyl, tert.Butyl oder Phenyl steht,
und
- n: - eine Zahl von 0 bis 5 bedeutet,
- Z: - für Norbornyl steht oder
für eine Gruppe der Formel 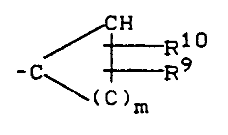 oder 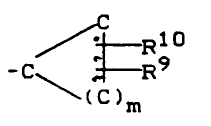 steht, wobei
- R⁹ und R¹⁰: gleich oder verschieden sind und Wasserstoff, Methyl, Ethyl, n-Propyl, iso-Propyl, Butyl oder tert.-Butyl bedeuten, oder
- R⁹ und R¹⁰: gemeinsam einen gesättigten carbocyclischen Ring mit bis zu 6 Kohlenstoffatomen bilden können und
- m: - eine Zahl von 1 bis 6 bedeutet,
- A und B: gleich oder verschieden sind und Wasserstoff, Methyl, Ethyl, Fluor oder Chlor bedeuten,

sowie deren Salze.

Besonders bevorzugte Verbindungen der allgemeinen Formel (I) sind solche, in denen
- R¹: - für eine Gruppe der Formel
-OR² oder 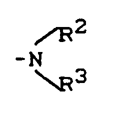 steht, wobei
- R² und R³: gleich oder verschieden sind und
- für Wasserstoff oder Methyl stehen, oder
- für eine Gruppe der Formel 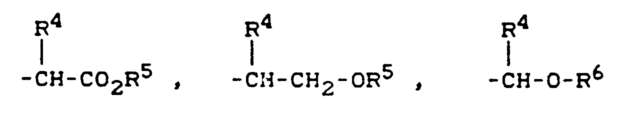 oder 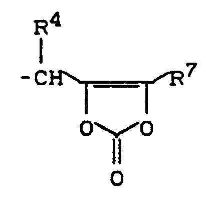 stehen, wobei
- R⁴: - für Wasserstoff, Methyl oder Phenyl steht,
- R⁵: - für Wasserstoff, Methyl, Ethyl, tert.-Butyl oder Benzyl steht,
- R⁶: - für eine Gruppe der Formel -COR⁵ steht, wobei R⁵ die oben angegebene Bedeutung hat, und
- R⁷: - für Methyl steht,
- Y: - für eine Gruppe der Formel 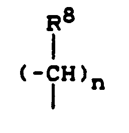 steht, wobei
- R⁸: - für Wasserstoff oder methyl steht,
und
- n: - eine Zahl 0 oder 1 bedeutet,
- Z: - für Norbornyl steht, oder
für eine Gruppe der Formel 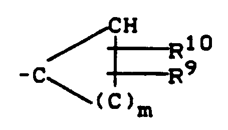 oder 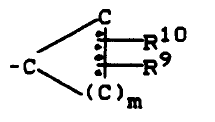 steht, wobei
- R⁹ und R¹⁰: gleich oder verschieden sind und Wasserstoff oder Methyl bedeuten, oder
- R⁹ und R¹⁰: gemeinsam einen Cyclohexylring bilden, und
- m: - eine Zahl 1, 2, 3, 4, oder 5 bedeutet,
- A und B: Wasserstoff oder Fluor bedeuten,
sowie deren Salze.

Die erfindungsgemäßen Verbindungen können in stereoisomeren Formen vorliegen, die sich entweder wie Bild und Spiegelbild (Enantiomere) oder die sich nicht wie Bild und Spiegelbild (Diastereomere) verhalten. Die Erfindung betrifft sowohl die Antipoden als auch die Racemformen sowie die Diastereomerengemische. Die Racemformen lassen sich ebenso wie die Diastereomeren in bekannter Weise in die stereoisomer einheitlichen Bestandteile trennen (vgl. E.L Eliel, Stereochemistry of Carbon Compounds, McGraw Hill, 1962).

Beispielsweise seien folgende Wirkstoffe im einzelnen genannt:
2-[4-(Chinolin-2-yl-methoxy)phenyl]-3-cyclopropylpropionsäuremethylester
2-[4-(Chinolin-2-yl-methoxy)phenyl]-3-cyclohexylpropionsäuremethylester
2-[4-(Chinolin-2-yl-methoxy)phenyl]-2-cyclopentylessigsäuremethylester
2-[4-(Chinolin-2-yl-methoxy)phenyl]-2-cyclohexylessigsäuremethylester
2-[4-(Chinolin-2-yl-methoxy)phenyl]-2-cycloheptylessigsäuremethylester
2-[4-(Chinolin-2-yl-methoxy)phenyl]-3-cyclopropylpropionsäure
2-[4-(Chinolin-2-yl-methoxy)phenyl]-3-cyclohexylpropionsäure
2-[4-(Chinolin-2-yl-methoxy)phenyl]-2-cyclopentylessigsäure
2-[4-(Chinolin-2-yl-methoxy)phenyl]-2-cyclohexylessigsäure
2-[4-(Chinolin-2-yl-methoxy)phenyl]-2-cycloheptylessigsäure
2-[4-(Chinolin-2-yl-methoxy)phenyl]-2-(cyclohex-2-enyl)essigsäuremethylester
2-[4-(Chinolin-2-yl-methoxy)phenyl]-2-cyclopropylpropionsäurebenzyloxycarbonylmethylester
2-[4-(Chinolin-2-yl-methoxy)phenyl]-2-cyclopentylessigsäurebenzyloxycarbonylmethylester
2-[4-(Chinolin-2-yl-methoxy)phenyl]-2-cyclopentylessigsäuremethyloxycarbonylmethylamid
2-[4-(Chinolin-2-yl-methoxy)phenyl]-2-(1-dekalinyl)essigsäuremethylester
2-[4-(Chinolin-2-yl-methoxy)phenyl]-2-cyclopentylessigsäure-tert.butyloxycarbonylmethylester
2-[4-(Chinolin-2-yl-methoxy)phenyl]-2-cyclopentylessigsäurepivaloyloxymethylester
2-[4-(Chinolin-2-yl-methoxy)phenyl]-2-cyclopentylessigsäuremethoxycarbonylmethylester
2-[4-(Chinolin-2-yl-methoxy)phenyl]-2-(1-dekalinyl)essigsäure
2-[4-(Chinolin-2-yl-methoxy)phenyl]-2-cyclopentylessigsäurecarboxymethylamid
2-[4-(Chinolin-2-yl-methoxy)phenyl]-2-cyclopentylessigsäure-Natriumsalz
2-[4-(Chinolin-2-yl-methoxy)phenyl]-3-cyclopentylpropionsäuremethylester
2-[4-(Chinolin-2-yl-methoxy)phenyl]-3-cyclopentylpropionsäure
2-[4-(Chinolin-2-yl-methoxy)phenyl]-2-(cyclohex-2-enyl) essigsäure
2-[4-(Chinolin-2-yl-methoxy)phenyl]-2-cyclopentylessigsäurecarboxymethylester
2-[4-(6-Fluorchinolin-2-yl-methoxy)phenyl-2-cyclopentylessigsäuremethylester
2-[4-(6-Fluorchinolin-2-yl-methoxy)phenyl-2-cyclopentylessigsäure
2-[4-(Chinolin-2-yl-methoxy)phenyl]-2-norbornylessigsäuremethylester
2-[4-(Chinolin-2-yl-methoxy)phenyl]-2-norbornylessigsäure
2-[4-(Chinolin-2-yl-methoxy)phenyl]-2-cyclopentylessigsäure[(L)-2-hydroxy-1-phenylethyl]amid (beide Diastereomere)
(+)-4-[2-(Chinolin-2-yl-methoxy)phenyl]-2-cyclopentylessigsäure
(-)-4-[2-(Chinolin-2-yl-methoxy)phenyl]-2-cyclopentylessigsäure

Außerdem wurden Verfahren zur Herstellung der Verbindungen der allgemeinen Formel (I) 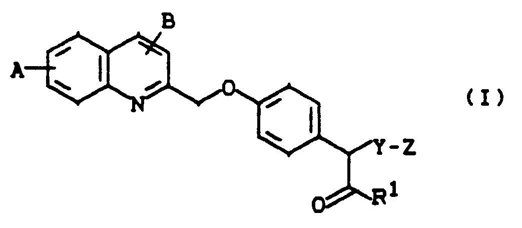
in welcher
A, B, R¹, Y und Z die oben angegebene Bedeutung haben,
gefunden,
die dadurch gekennzeichnet sind, daß man

### [A] 4-(Chinolin-2-yl-methoxy)phenylessigsäureester der allgemeinen Formel (Ia)

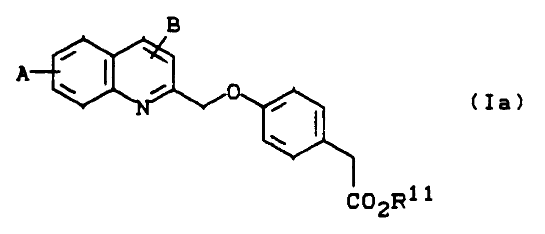

in welcher
- R¹¹: - für Alkyl steht und
A und B die oben angegebene Bedeutung haben,
mit Verbindungen der allgemeinen Formel (II)

Y-Z-X (II)

in welcher
Y und Z die oben angegebene Bedeutung haben, und
- X: - für Chlor, Brom oder Iod steht,
alkyliert und im Falle der Säuren die Ester verseift, oder indem man

### [B] die Säuren der allgemeinen Formel (Ib)

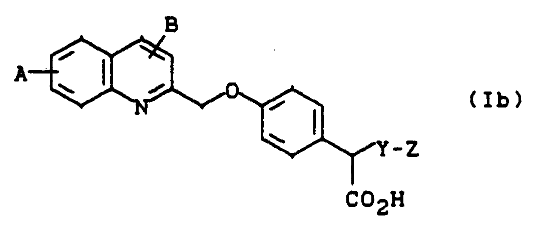

in welcher
A, B, Y und Z die oben angegebene Bedeutung haben,
mit Verbindungen der allgemeinen Formel (III)

X-R¹² (III)

in welcher
- R¹²: - für eine Gruppe der Formel 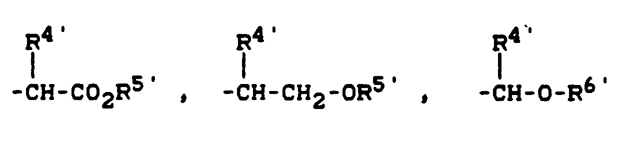 oder 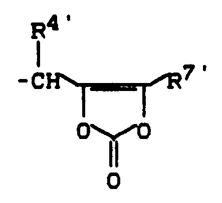 steht, wobei
- R^{4′}: - für Alkyl, Aralkyl oder Aryl steht, das gegebenenfalls substituiert sein kann durch Hydroxy, Carboxyl, Alkoxycarbonyl, Alkylthio, Heteroaryl oder Aminocarbonyl,
- R^{5′}: - für Alkyl, Aryl oder Aralkyl steht,
- R^{6′}: - für eine Gruppe der Formel -COR^{5′} oder -CO₂R^{5′}steht, wobei
R^{5′} die oben angegebene Bedeutung hat
- R⁷: ^{′} - für Alkyl oder Aryl steht,
und
X die oben angegebene Bedeutung hat,
verestert und im Fall der Säuren die Ester einer hydrogenolytischen Spaltung unterzieht,
oder indem man

### [C] die Säuren der allgemeinen Formel (Ib) mit Aminen der allgemeinen Formel (IV)

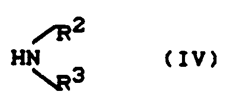

in welcher
R² und R³ die oben angegebene Bedeutung haben,
mit der Maßgabe, daß R⁵ nicht Wasserstoff bedeutet, wenn R² oder R³ für die Gruppe 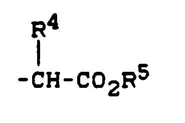
steht wobei
R⁴ und R⁵ die obengenannte Bedeutung haben,
in Gegenwart von üblichen Aktivierungsreagentien amidiert und im Fall der Säuren die Ester verseift
oder indem man

### [D] Phenole der allgemeinen Formel (V)

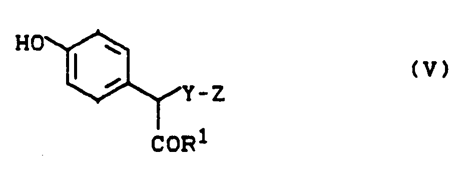

in welcher
R¹, Y und Z die oben angegebene Bedeutung haben
mit
2-Halogenmethylchinolinen der Formel (VI) 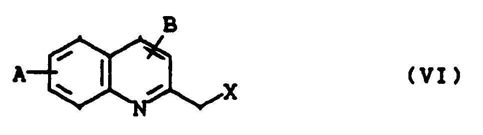
in welcher
A, B und X die oben angegebene Bedeutung haben,
verethert und im Fall der Säuren die Ester verseift.

Die erfindungsgemäßen Verfahren können durch die folgenden Formelschemen erläutert werden:

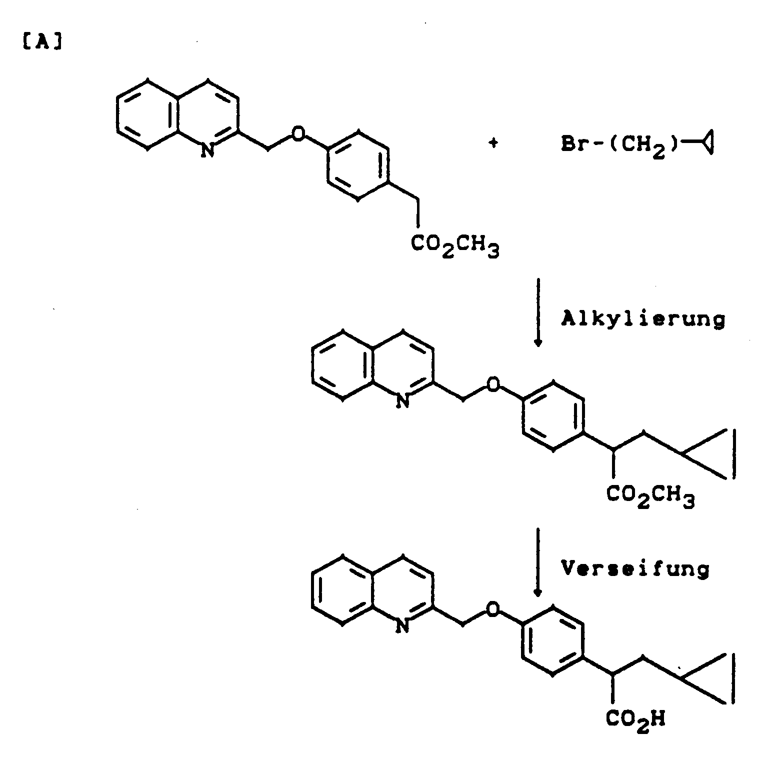

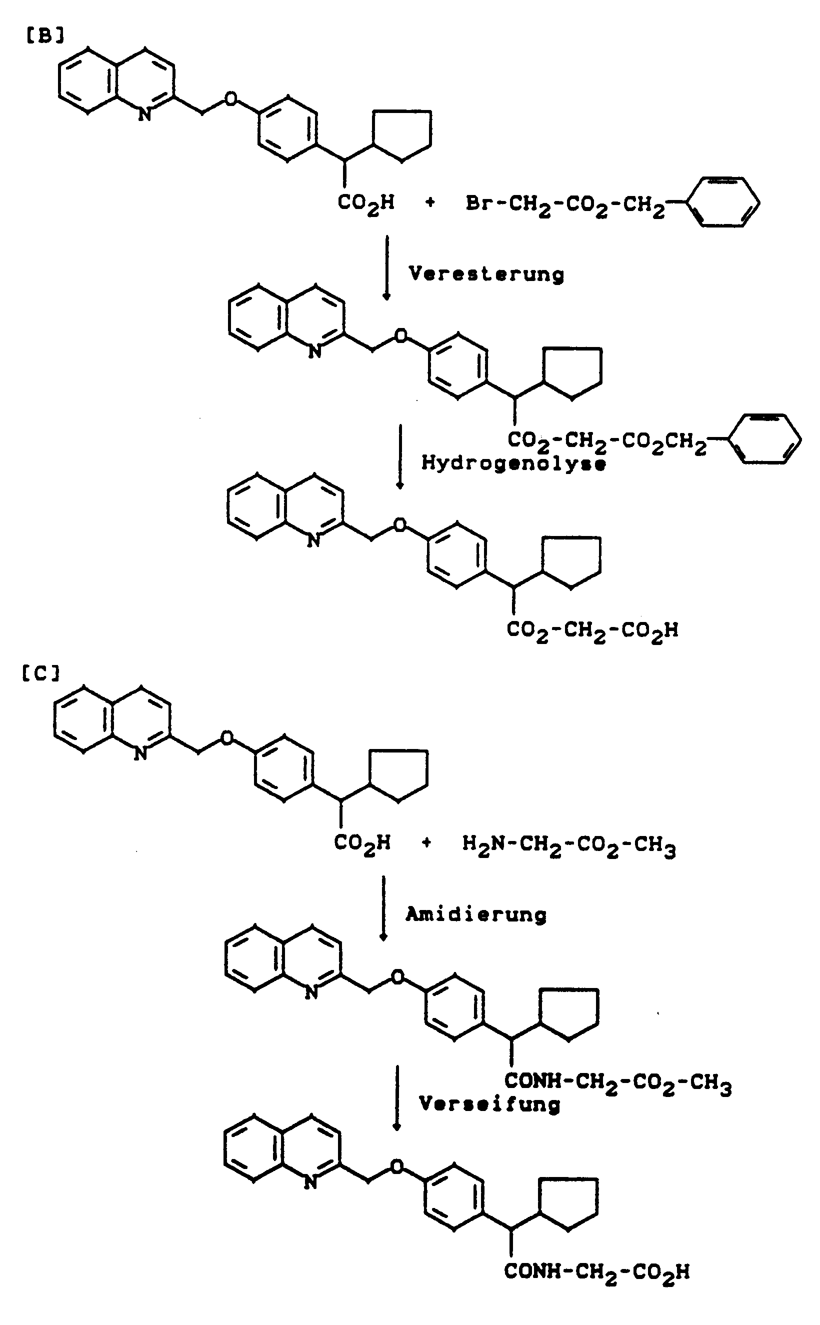

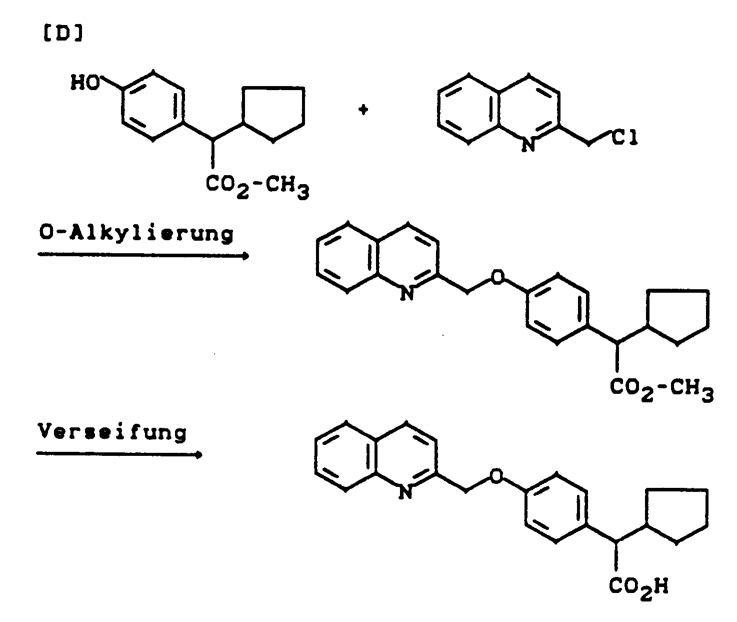

Die Alkylierung der C-H aciden Verbindungen (Formel Ia) mit Alkylhalogeniden erfolgt im allgemeinen in inerten Lösemitteln in Anwesenheit einer Base.

Als Lösemittel eignen sich hierbei, je nach Art des Alkylierungsmittels alle inerten organischen Lösemittel. Hierzu gehören bevorzugt Ether wie Diethylether, Dioxan oder Tetrahydrofuran, oder Kohlenwasserstoffe wie Benzol, Toluol oder Xylol, oder Dimethylformamid oder Hexamethylphosphorsäuretriamid, oder Gemische der genannten Lösemittel.

Als Basen eignen sich die üblichen basischen Verbindungen. Hierzu gehören bevorzugt Alkalihydride wie Natriumhydrid, Alkaliamide wie Natriumamid oder Lithiumdiisopropylamid, Alkalialkoholate wie Natriummethanolat, Natriumethanolat, Kaliummethanolat, Kaliumethanolat oder Kalium-tert.butylat, oder organische Amine wie Trialkylamine z.B. Triethylamin, oder lithiumorganische Verbindungen wie Butyllithium oder Phenyllithium.

Die Alkylierung der CH-aciden Verbindungen erfolgt im allgemeinen in einem Temperaturbereich von 0°C bis 150°C, bevorzugt von 10°C bis 100°C.

Die Alkylierung der CH-aciden Verbindungen wird im allgemeinen bei Normaldruck durchgeführt. Es ist aber auch möglich, das Verfahren bei Unterdruck oder Überdruck durchzuführen (z.B. in einem Bereich von 0,5 bis 5 bar).

Im allgemeinen setzt man 0,5 bis 5, bevorzugt 1 bis 2 mol Halogenid, bezogen auf 1 mol des Reaktionspartners ein. Die Base wird im allgemeinen in einer Menge von 0,5 bis 5 mol, bevorzugt von 1 bis 3 mol bezogen auf das Halogenid eingesetzt.

Die Verseifung der Carbonsäureester erfolgt nach üblichen Methoden, indem man die Ester in inerten Lösemitteln mit üblichen Basen behandelt, wobei die zunächst entstehenden Salze durch Behandeln mit Säure in die freien Carbonsäuren überführt werden können.

Als Basen eignen sich für die Verseifung die üblichen anorganischen Basen. Hierzu gehören bevorzugt Alkalihydroxide oder Erdalkalihydroxide wie beispielsweise Natriumhydroxid, Kaliumhydroxid oder Bariumhydroxid, oder Alkalicarbonate wie Natrium- oder Kaliumcarbonat oder Natriumhydrogencarbonat, oder Alkalialkoholate wie Natriumethanolat, Natriummethanolat, Kaliumethanolat, Kaliummethanolat oder Kalium-tert.butanolat. Besonders bevorzugt werden Natriumhydroxid oder Kaliumhydroxid eingesetzt.

Als Lösemittel eignen sich für die Verseifung Wasser oder die für eine Verseifung üblichen organischen Lösemittel. Hierzu gehören bevorzugt Alkohole wie Methanol, Ethanol, Propanol, Isopropanol oder Butanol, oder Ether wie Tetrahydrofuran oder Dioxan, oder Dimethylformamid oder Dimethylsulfoxid. Besonders bevorzugt werden Alkohole wie Methanol, Ethanol, Propanol oder Isopropanol verwendet. Ebenso ist es möglich, Gemische der genannten Lösemittel einzusetzen.

Die Verseifung wird im allgemeinen in einem Temperaturbereich von 0°C bis +100°C, bevorzugt von +20°C bis +80°C durchgeführt.

Im allgemeinen wird die Verseifung bei Normaldruck durchgeführt. Es ist aber auch möglich, bei Unterdruck oder bei Überdruck zu arbeiten (z.B. von 0,5 bis 5 bar).

Bei der Durchführung der Verseifung wird die Base im allgemeinen in einer Menge von 1 bis 3 mol, bevorzugt von 1 bis 1,5 mol bezogen auf 1 mol des Esters bzw. des Lactons eingesetzt. Besonders bevorzugt verwendet man molare Mengen der Reaktanden.

Bei der Durchführung der Verseifung entstehen im ersten Schritt die Salze der erfindungsgemäßen Verbindungen als Zwischenprodukte, die isoliert werden können. Die erfindungsgemäßen Säuren erhält man durch Behandeln der Salze mit üblichen anorganischen Säuren. Hierzu gehören bevorzugt Mineralsäuren wie beispielsweise Chlorwasserstoffsäure, Bromwasserstoffsäure, Schwefelsäure oder Phosphorsäure. Es hat sich bei der Herstellung der Carbonsäuren hierbei als vorteilhaft erwiesen, die basische Reaktionsmischung der Verseifung in einem zweiten Schritt ohne Isolierung der Salze anzusäuern. Die Säuren können dann in üblicher Weise isoliert werden.

Die Veresterung der Carbonsäuren erfolgt nach üblichen Methoden, indem man die Säuren in inerten Lösemitteln, gegebenenfalls in Anwesenheit einer Base, mit Alkylhalogeniden behandelt.

Als Basen eignen sich die üblichen organischen Amine. Hierzu gehören bevorzugt Alkylamine wie Triethylamin, Diisopropylamin, Dicyclohexylamin und Ethyldiisopropylamin.

Als Lösemittel eignen sich hierbei alle inerten organischen Lösemittel. Hierzu gehören bevorzugt Ether wie Diethylether, Dioxan oder Tetrahydrofuran, oder Kohlenwasserstoffe wie Benzol, Toluol oder Xylol, oder Dimethylformamid oder Gemische der genannten Lösemittel.

Die Veresterung der Carbonsäuren erfolgt im allgemeinen in einem Temperaturbereich von 0°C bis 150°C, bevorzugt von 10°C bis 100°C.

Die Veresterung der Carbonsäuren wird im allgemeinen bei Normaldruck durchgeführt. Es ist aber auch möglich, das Verfahren bei Unterdruck oder Überdruck durchzuführen (z.B. in einem Bereich von 0,5 bis 5 bar).

Im allgemeinen setzt man 0,5 bis 5, bevorzugt 1 bis 2 mol Halogenid, bezogen auf 1 mol des Reaktionspartners ein. Die Base wird im allgemeinen in einer Menge von 0,5 bis 5 mol, bevorzugt von 1 bis 3 mol bezogen auf das Halogenid eingesetzt.

Im allgemeinen setzt man 0,01 bis 1, bevorzugt 0,05 bis 0,5 mol Katalysator, bezogen auf 1 mol Reaktionspartner ein.

Die hydrogenolytische Spaltung der Benzylester erfolgt nach üblichen Methoden, indem man die Benzylester in einem inerten Lösemittel in Anwesenheit eines Katalysators mit Wasserstoff-Gas hydriert.

Als Katalysatoren eignen sich die üblichen Metallkatalysatoren, die gegebenenfalls auf einem inerten Träger wie zum Beispiel Kohle in variablen Konzentrationen aufgebracht sind. Hierzu gehören bevorzugt Palladium, Nickel, Platin, besonders bevorzugt 5 bis 15% Palladium auf Aktivkohle.

Als Lösemittel eignen sich hierbei alle inerten organischen Lösemittel. Hierzu gehören bevorzugt Ether wie Diethylether, Dioxan oder Tetrahydrofuran, oder Kohlenwasserstoffe wie Benzol, Toluol oder Xylol, oder Alkohole wie Methanol, Ethanol oder Propanol, oder niedrigsiedende Ester wie Essigester, oder Amine wie Triethylamin, oder Gemische der genannten Lösemittel.

Die hydrogenolytische Spaltung erfolgt im allgemeinen in einem Temperaturbereich von 0°C bis 150°C, bevorzugt von 10°C bis 100°C.

Die hydrogenolytische Spaltung wird im allgemeinen mit Wasserstoff bei Normaldruck durchgeführt. Es ist aber auch möglich, das Verfahren bei Überdruck durchzuführen (z.B. in einem Bereich von 1 bis 10 bar).

Im allgemeinen setzt man 0,01 bis 1, bevorzugt 0,05 bis 0,5 mol Katalysator, bezogen auf 1 mol Reaktionspartner ein.

Die Amidierung der Verbindungen (Ib) mit Aminen erfolgt im allgemeinen in inerten Lösemitteln in Anwesenheit einer Base.

Als Lösemittel eignen sich hierbei je nach Art des Amins alle inerten organischen Lösemittel. Hierzu gehören bevorzugt Ether wie Diethylether, Dioxan oder Tetrahydrofuran, Kohlenwasserstoffe wie Benzol, Toluol oder Xylol, oder Dimethylformamid oder Gemische der genannten Lösemittel. Besonders bevorzugt ist Dimethylformamid.

Als Basen eignen sich die üblichen basischen Verbindungen. Hierzu gehören bevorzugt organische Amine wie Trialkylamine z.B. Triethylamin.

Als Aminkomponenten eignen sich neben den üblichen Aminen wie beispielsweise Propylamin, Dimethylamin oder Diethylamin auch optisch aktive Aminosäureester wie beispielsweise die Ester von Alanin, Leucin, Methionin, Threonin, Tyrosin, Cystin, Glycin, Isoleucin, Lysin, Phenylalanin, Phenylglycin oder Valin, oder Aminoalkohole wie beispeilsweise 2-Aminoethanol oder Phenylglycinol/alamin, wobei letzterer in optische reiner Form durch Reduktion der entsprechenden Aminosäure nach bekannter Methode dargestellt werden kann (vgl. G.C. Barrett, Chemistry and Biochemistry of the Amino Acids, Chapman and Hall, 1985).

Durch Einsatz der oben genannten Aminkomponenten können somit in Analogie zu dem oben beschriebenen Verfahren [C] die diastereomeren Amide der Verbindungen der Formel (I) hergestellt werden. Nach Trennung der Diastereomere nach den oben aufgeführten üblichen Methoden und anschließender Verseifung werden die reinen Enantiomeren der erfindungsgemäßen Verbindungen der Formel (I) erhalten.

Als Aktivierungsreagentien werden im allgemeinen die üblichen Peptidkupplungsreagentien verwendet. Hierzu gehören bevorzugt Carbodiimide wie beispielsweise Diisopropylcarbodiimid, Dicyclohexylcarbodiimid oder N-(3-Dimethylaminoisopropyl)-N′-ethylcarbodiimid Hydrochlorid oder Carbonylverbindungen wie Carbonyldiimidazol oder 1,2-Oxazoliumverbindungen wie 2-Ethyl-5-phenyl-1,2-oxazolium-3-sulfonat oder Propanphosphonsäureanhydrid oder Isobutylchloroformat oder Benzotriazolyloxy-tris-(dimethylamino)-phosphonium-hexafluorophosphat oder Phosphorsäurediphenylesteramid oder Methansulfonsäurechlorid, gegebenenfalls in Anwesenheit von Basen wie Triethylamin oder N-Ethylmorpholin oder N-Methylpiperidin oder Dicyclohexylcarbodiimid und N-Hydroxysuccinimid.

Die Hydrolyse erfolgt im allgemeinen mit anorganischen oder organischen Säuren wie Salzsäure, Bromwasserstoffsäure, Schwefelsäure, Phosphorsäure, Ameisensäure, Essigsäure, Propionsäure, Methansulfonsäure oder Trifluoressigsäure oder Gemischen der genannten Säuren.

Die Amidierung der erfindungsgemäßen Verbindungen der allgemeinen Formel (I) erfolgt im allgemeinen in einem Temperaturbereich von 0°C bis +150°C, bevorzugt von 0°C bis +50°C.

Die Amidierung wird im allgemeinen bei Normaldruck durchgeführt. Es ist aber auch möglich, das Verfahren bei Unterdruck oder bei Überdruck durchzuführen (z.B. in einem Bereich von 0,5 bis 5 bar).

Die als Ausgangsverbindungen verwendeten Ester der allgemeinen Formel (Ia) werden auch aus den bekannten 4-Hydroxyphenylessigsäuren durch Veretherung mit 2-Halogenmethylchinolinen der allgemeinen Formel (VI) analog Verfahren D hergestellt.

Die Veretherung kann in inerten organischen Lösemitteln gegebenenfalls in Anwesenheit einer Base durchgeführt werden.

Lösemittel für die Veretherung können inerte organische Lösemittel sein, die sich unter den Reatkionsbedingungen nicht verändern, Hierzu gehören vorzugsweise Ether wie beispielsweise Dioxan, Tetrahydrofuran oder Diethylether, Halogenkohlenwasserstoffe wie Dichlormethan, Trichlormethan, Tetrachlormethan, 1,2-Dichlorethan oder Trichlorethylen, Kohlenwasserstoffe wie Benzol, Xylol, Toluol, Hexan, Cyclohexan oder Erdölfraktionen, Nitromethan, Dimethylformamid, Acetonitril, Aceton oder Hexamethylphosphorsäuretriamid. Ebenso ist es möglich, Gemische der Lösemittel einzusetzen.

Als Basen für die Veretherung können anorganische oder organische Basen eingesetzt werden. Hierzu gehören vorzugsweise Alkalihydroxide wie zum Beispiel Natriumhydroxid oder Kaliumhydroxid, Erdalkalihydroxide wie zum Beispiel Bariumhydroxid, Alkalicarbonate wie Natriumcarbonat oder Kaliumcarbonat, Erdalkalicarbonate wie Calciumcarbonat, oder organische Amine (Trialkyl(C₁-C₆)amine) wie Triethylamin, oder Heterocyclen wie Pyridin, Methylpiperidin, Piperidin oder Morpholin.

Es ist auch möglich, als Basen Alkalimetalle wie Natrium, und deren Hydride, wie Natriumhdyrid einzusetzen.

Die Veretherung zur Darstellung der Verbindungen der Formel (Ia) erfolgt im allgemeinen in einem Temperaturbereich von 0°C bis 150°C, bevorzugt von 10°C bis 100°C, und im allgemeinen bei Normaldruck. Es ist aber auch möglich, das Verfahren bei Unterdruck oder Überdruck durchzuführen (z.B. in einem Bereich von 0,5 bis 5 bar).

Im allgemeinen setzt man 0,5 bis 5, bevorzugt 1 bis 2 mol Halogenid, bezogen auf 1 mol des Reaktionspartners ein. Die Base wird im allgemeinen in einer Menge von 0,5 bis 5 mol, bevorzugt von 1 bis 3 mol bezogen auf das Halogenid eingesetzt.

4-Hydroxyphenylessigsäureester sind bekannt oder können nach üblichen Methoden aus den entsprechenden Phenolen unter Abspaltung von geeigneten Schutzgruppen hergestellt werden [vgl. H. Beyer, Lehrbuch der organischen Chemie, S. Hirzel Verlag, Stuttgart; Protective Groups in Organic Synthesis, J. Wiley & Sons, 1981, New York].

Die substituierten 4-Hydroxyphenylessigsäureester der allgemeinen Formel (V) sind größtenteils neu und können aus den oben erwähnten 4-Hydroxyphenylessigsäureestern durch Alkylierung nach bekannter Methode dargestellt werden (vgl. Ferri, Reaktionen der organischen Synthese, Georg Thieme Verlag, Stuttgart, 1978).

2-Halogenmethylchinoline der Formel (VI) wie beispielsweise 2-Chlormethylchinolin, sind bekannt und können nach üblichen Methoden hergestellt werden [vgl. Chem. Berichte. 120, 649, 1987].

Die Verbindungen der Formel (II) und (III) sind bekannt oder können nach üblichen Halogenisierungsmethoden hergestellt werden [vgl. Organikum VEB Deutscher Verlag der Wissenschaften, Berlin 1977].

Die erfindungsgemäßen Säuren, Ester und Amide können als Wirkstoffe in Arzneimitteln eingesetzt werden. Die Stoffe wirken besonders als Hemmer von enzymatischen Reaktionen im Rahmen des Arachidonsäurestoffwechsels, insbesondere der 5-Lipoxygenase.

Die erfindungsgemäßen Verbindungen zeigen nach oraler Gabe in Lipoxygenase-sensitiven Testmodellen eine gute Wirkung.

Sie sind somit bevorzugt zur Behandlung und Verhütung von Erkrankungen der Atemwege wie Allergien/Asthma, Bronchitis, Emphysen, Schocklunge, pulmonaler Hypertonie, Entzündungen/Rheuma und Ödemen, Thrombosen und Thromboembolien, Ischämien (periphere, cardiale, cerebrale Durchblutungsstörungen), Herz- und Hirninfarkten, Herzrhythmusstörungen, Angina pectoris, Arteriosklerose, bei Gewebstransplantationen, Dermatosen wie Psoriasis, Metastasen und zur Cytoprotection im Gastrointestinaltrakt geeignet.

Die neuen Wirkstoffe können in an sich bekannter Weise unter Verwendung inerter nichttoxischer, pharmazeutischer geeigneter Trägerstoffe oder Lösungsmittel in die üblichen Formulierungen überführt werden, wie Tabletten, Kapseln, Dragees, Pillen, Granulate, Aerosole, Sirupe, Emulsionen, Suspensionen und Lösungen. Hierbei soll die therapeutisch wirksame Verbindung jeweils in einer Konzentration von etwa 0,5 bis 90 Gew.-%, bevorzugt von 10 bis 70 Gew.-%, in der Gesamtmischung vorhanden sein, d.h. in Mengen, die ausreichend sind, um den angegebenen Dosierungsspielraum zu erreichen.

Die Formulierungen werden beispielsweise hergestellt durch Vestrecken der Wirkstoffe mit Lösungsmitteln und/oder Trägerstoffen, gegebenenfalls unter Verwendung von Emulgiermitteln und/oder Dispergiermitteln, wobei z.B. im Fall der Benutzung von Wasser als Verdünnungsmittel gegebenenfalls organische Lösungsmittel als Hilfslösungsmittel verwendet werden können.

Als Hilfsstoffe seien beispielsweise aufgeführt: Wasser, nichttoxische organische Lösungsmittel wie Paraffine (z.B. Erdölfraktionen), pflanzliche Öle (z.B. Erdnuß/Sesamöl), Alkohole (z.B. Ethylalkohol, Glycerin), Glykole (z.B. Propylenglykol, Polyethylenglykol), feste Trägerstoffe, wie natürliche Gesteinsmehle (z.B. Kaoline, Tonerden, Talkum, Kreide), synthetische Gesteinsmehle (z.B. hochdisperse Kieselsäure, Silikate), Zucker (z.B. Rohr-, Milch- und Traubenzucker), Emulgiermittel (z.B. Polyoxyethylen-Fettsäureester, Polyoxyethylen-Fettalkohole-Ether, Alkylsulfonate und Arylsulfonate), Dispiergiermittel (z.B. Lignin-Sulfitablaugen, Methylcellulose, Stärke und Polyvinylpyrrolidon) und Gleitmittel (z.B. Magnesiumstearat, Talkum, Stearinsäure und Natriumlaurylsulfat.

Die Applikation kann in üblicher Weise erfolgen, vorzugsweise oral oder parenteral, insbesondere perlingual oder intravenös. Im Falle der oralen Anwendung können Tabletten selbstverständlich außer den genannten Trägerstoffen auch Zusätze wie Natriumcitrat, Calciumcarbonat und Dicalciumphosphat zusammen mit verschiedenen Zuschlagstoffen wie Stärke, vorzugsweise Kartoffelstärke, Gelatine und dergleichen enthalten. Weiterhin können Gleitmittel wie Magnesiumstearat, Natriumlaurylsulfat und Talkum zum Tablettieren mitverwendet werden. Im Falle wäßriger Suspensionen und/oder Elixieren, die für orale Anwendungen gedacht sind, können die Wirkstoffe außer den oben genannten Hilfsstoffen mit verschiedenen Geschmacksaufbesserern oder Farbstoffen versetzt werden.

Für den Fall der parenteralen Anwendungen können Lösungen der Wirkstoffe unter Verwendung geeigneter flüssiger Trägermaterialien eingesetzt werden.

Im allgemeinen hat es sich als vorteilhaft erwiesen, bei intravenöser Applikation Mengen von etwa 0,01 bis 10 mg/kg, vorzugsweise 0,01 bis 5 mg/kg Körpergewicht zur Erzielung wirksamer Ergebnisse zu verabreichen. Bei oraler Applikation beträgt die Dosierung im allgemeinen etwa 0,1 bis 200 mg/kg, vorzugsweise 1 bis 100 mg/kg Körpergewicht.

Trotzdem kann es gegebenenfalls erforderlich sein, von den genannten Mengen abzuweichen, und zwar in Abhängigkeit vom Körpergewicht bzw. der Art des Applikationsweges, vom individuellen Verhalten gegenüber dem Medikament, der Art von dessen Formulierung und zu dem Zeitpunkt bzw. Intervall, zu welchem die Verabreichung erfolgt. So kann es in einigen Fällen ausreichend sein, mit weniger als der vorgenannten Mindestmenge auszukommen, während in anderen Fällen die genannte obere Grenze überschritten werden muß. Im Fall der Applikation größerer Mengen kann es empfehlenswert sein, diese in mehreren Einzelgaben über den Tag zu verteilen.

Die erfindungsgemäßen Säuren und Ester können sowohl in der Humanmedizin als auch in der Veterinärmedizin angewendet werden.

### Herstellungsbeispiele

### Beispiel 1 (Ausgangsverbindung)

### 4-(Chinolin-2-yl-methoxy)phenylessigsäuremethylester

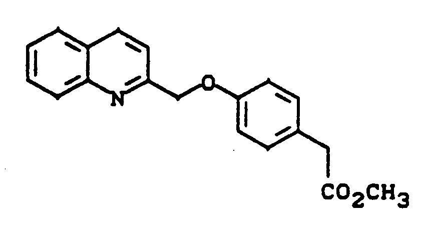

200 g (1,2 mol) 4-Hydroxyphenylessigsäuremethylester und 166 g (1,2 mol) Kaliumcarbonat werden in 2 l Dimethylformamid 1 h bei 25°C gerührt. Nach Zugabe von 214 g (1,2 mol) 2-Chlormethylchinolin wird 15 h auf 50°C erwärmt. Nach Einengen im Vakuum wird der Rückstand zwischen Wasser und Essigester verteilt, die organische Phase über Natriumsulfat getrocknet und eingeengt. Das verbleibende Produkt wird aus Methanol umkristallisiert.
Ausbeute: 293 g (79% der Theorie)
Festpunkt: 71 - 73°C

### Beispiel 2

### 2-[4-(Chinolin-2-yl-methoxy)phenyl]-3-cyclopropylpropionsäuremethylester

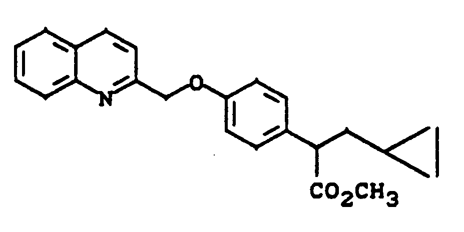

Zu einer Suspension von 1,5 g (55 mmol) Natriumhydrid in 60 ml Dimethylformamid werden unter Schutzgas bei 0°C 15,4 g (50 mmol) 4-(Chinolin-2-yl-methoxy)phenylessigsäuremethylester zugetropft. Nach beendeter Wasserstoffentwicklung wird 1 h bei 25°C nachgerührt, unter Eiskühlung 7,4 g (55 mmol) (Brommethyl)-cyclopropan in 60 ml Dimethylformamid zugetropft und 16 h bei 25°C gerührt. Nach Abdampfen des Lösemittels im Vakuum wird der Rückstand zwischen Essigester und Wasser verteilt, die organische Phase über Natriumsulfat getrocknet und eingeengt. Der Rückstand wird aus Methanol umkristallisiert.
Ausbeute: 15 g (83% der Theorie)
Festpunkt: 47°C

### Beispiel 3

### 2-[4-(Chinolin-2-yl-methoxy)phenyl]-3-cyclohexylpropionsäuremethylester

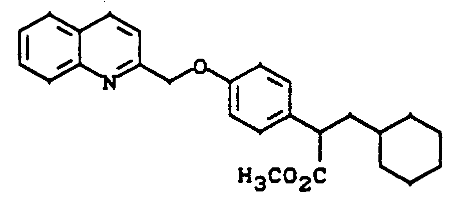

Die Darstellung erfolgt aus 15,4 g (50 mmol) 4-(Chinolin-2-yl-methoxy)phenylessigsäuremethylester und 9,74 g (55 mmol) (Brommethyl)-cyclohexan analog der Vorschrift von Beispiel 2.
Ausbeute: 15,9 g (79% der Theorie)
Festpunkt: 69°C

### Beispiel 4

### 2-[4-(Chinolin-2-yl-methoxy)phenyl]-2-cyclopentylessigsäuremethylester

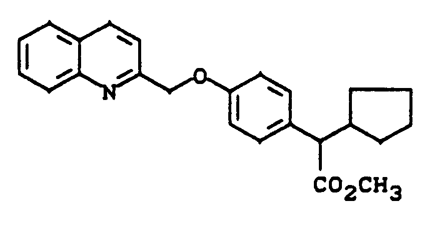

### Verfahren a)

Die Darstellung erfolgt aus 15,4 g (50 mmol) 4-(Chinolin-2-yl-methoxy)phenylessigsäuremethylester und 8,2 g (55 mmol) Cyclopentylbromid analog der Vorschrift von Biespiel 2.
Ausbeute: 12,8 g (68% der Theorie)
Festpunkt: 94°C

### Verfahren b)

2,3 g (10 mmol) 2-(Cyclopentyl-2(4-hydroxyphenyl)-essigsäuremethylester werden in 30 ml Dimethylformamid gelöst. Nach Zugabe von 1,4 g (10 mmol) Kaliumcarbonat wird 1 h bei 60°C gerührt, eine Lösung von 2,3 g (10 mmol) 2-Chlormethylchinolin in 20 ml Dimethylformamid zugetropft und 15 h bei 60°C gerührt. Nach Abkühlen wird eingeengt, der Rückstand in Essigester aufgenommen und zweimal mit Wasser gewaschen. Nach Trocknen über Natriumsulfat wird eingeengt und der Rückstand aus Methanol unkristallisiert.
Ausbeute: 3,18 g (85% der Theorie)

### Beispiel 5

### 2-[4-(Chinolin-2-yl-methoxy)phenyl]-2-cyclohexylessigsäuremethylester

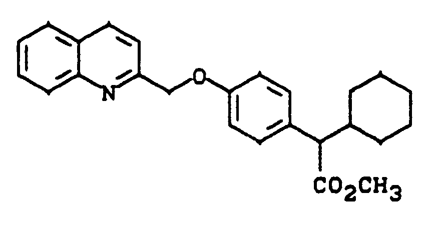

Die Darstellung erfolgt aus 15,4 g (50 mmol) 4-(Chinolin-2-yl-methoxy)phenylessigsäuremethylester und 11,55 g (55 mmol) Cyclohexyliodid analog der Vorschrift von Beispiel 2.
Ausbeute: 11,74 g (60% der Theorie)
Festpunkt: 92°C

### Beispiel 6

### 2-[4-(Chinolin-2-yl-methoxy)phenyl]-2-cycloheptylessigsäuremethylester

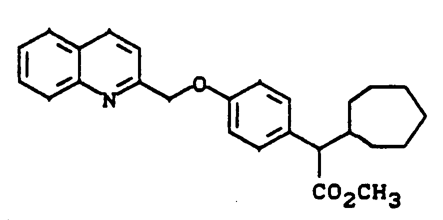

Die Darstellung erfolgt aus 15,4 g (50 mmol) 4-(Chinolin-2-yl-methoxy)phenylessigsäuremethylester und 9,07 g (55 mmol) Cycloheptylbromid analog der Vorschrift von Beispiel 2.
Ausbeute: 16 g (80% der Theorie)
Festpunkt: 81°C

### Beispiel 7

### 2-[4-(Chinolin-2-yl-methoxy)phenyl]-3-cyclopropylpropionsäure

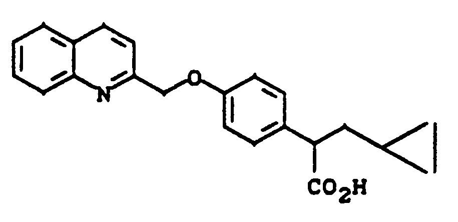

13,33 g (37 mmol) 2-[4-(Chinolin-2-yl-methoxy)phenyl]-3-cyclopropyl-propionsäuremethylester werden in 200 ml Methanol und 55,4 ml 1 molarer Natriumhydroxydlösung 10 h auf Rückfluß erwärmt. Nach Abkühlen wird mit konzentrierter Salzsäure angesäuert, das ausgefallene Produkte abgesaugt und getrocknet.
Ausbeute: 12,5 g (98% der Theorie)
Festpunkt: 146°C

### Beispiel 8

### 2-[4-(Chinolin-2-yl-methoxy)phenyl]-3-cyclohexylpropionsäure

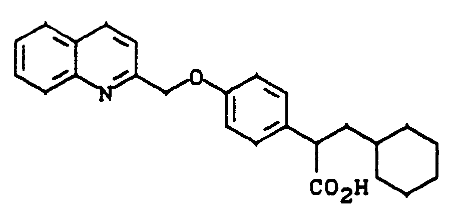

Die Darstellung erfolgt aus 6,25 g (15,5 mmol) 2-[4-(Chinolin-2-yl-methoxy)phenyl]-3-cyclohexyl-propionsäuremethylester analog der Vorschrift von Beispiel 7.
Ausbeute: 5 g (83% der Theorie)
Festpunkt: 148 - 151°C

### Beispiel 9

### 2-[4-(Chinolin-2-yl-methoxy)phenyl]-2-cyclopentylessigsäure

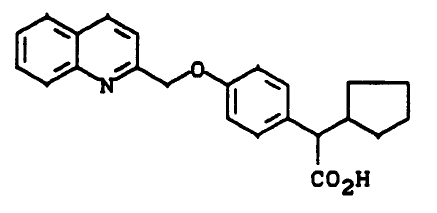

Die Darstellung erfolgt aus 10,87 g (29 mmol) 2-[4-(Chinolin-2-yl-methoxy)phenyl]-2-cyclopentyl-essigsäuremethylester analog der Vorschrift von Beispiel 7.
Ausbeute: 8,8 g (84% der Theorie)
Festpunkt: 183 - 185°C

### Biespiel 10

### 2-[4-(Chinolin-2-yl-methoxy)phenyl]-2-cyclohexylessigsäure

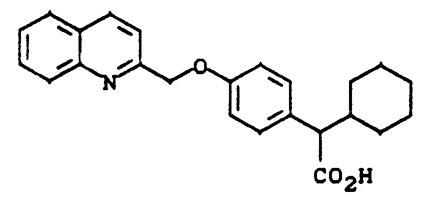

Die Darstellung erfolgt aus 10 g (26 mmol) 2-[4-(Chinolin-2-yl-methoxy)phenyl]-2-cyclohexyl-essigsäuremethylester analog der Vorschrift von Beispiel 7.
Ausbeute: 8,7 g (90% der Theorie)
Festpunkt: 201 - 207°C

### Beispiel 11

### 2-[4-(Chinolin-2-yl-methoxy)phenyl]-2-cycloheptylessigsäure

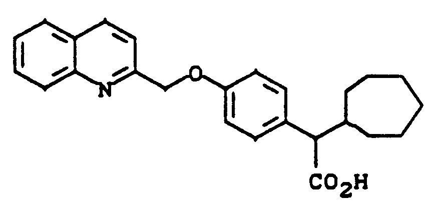

Die Darstellung erfolgt aus 11 g (27 mmol) 2-[4-(Chinolin-2-yl-methoxy)phenyl]-2-cycloheptyl-essigsäuremethylester analog der Vorschrift von Beispiel 7.
Ausbeute: 9,3 g (87% der Theorie)
Festpunkt: 176°C

### Beispiel 12

### 2-[4-(Chinolin-2-yl-methoxy)phenyl]-2-(cyclohex-2-enyl)essigsäuremethylester

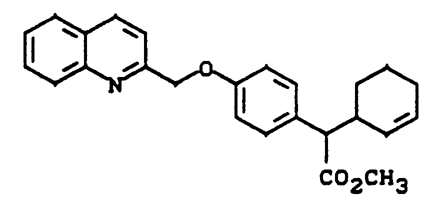

Die Darstellung erfolgt aus 15,4 g (50 mmol) 4-(Chinolin-2-yl-methoxy)phenylessigsäuremethylester und 8,86 g (55 mmol) 3-Bromcyclohexen analog der Vorschrift von Beispiel 2.
Ausbeute: 14,74 g (76% der Theorie)
Festpunkt: 102 - 104°C

### Beispiel 13

### 2-[4-(Chinolin-2-yl-methoxy)phenyl]-3-cyclopropyl-propionsäurebenzyloxycarbonylmethylester

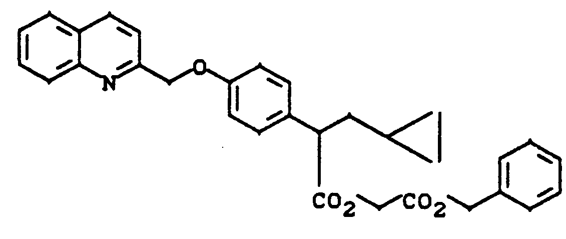

7 g 2-[4-(Chinolin-2-yl-methoxy)phenyl]-3-cyclopropylpropionsäure, 5 g Bromessigsäurebenzylester und 4 g Dicyclohexylamin werden in 100 ml Tetrahydrofuran 15 h unter Rückfluß erwärmt. Nach Abkühlen auf 0°C wird vom ausgefallenen Salz abfiltriert und das Lösemittel im Vakuum abgedampft. Der Rückstand wird an Kieselgel mit Methylenchlorid chromatographiert. Man erhält ein Öl.
Ausbeute: 9,27 g (93% der Theorie)
Rₜ (HPLC) = 4,30 min (RP 8, 7 µm; Acetonitril/Wasser (70:30)).

### Beispiel 14

### 2-[4-(Chinolin-2-yl-methoxy)phenyl]-2-cyclopentyl-essigsäurebenzyloxycarbonylmethylester

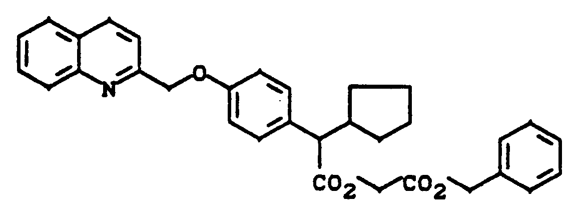

Die Darstellung erfolgt aus 7,22 g (20 mmol) 2-[4-(Chinolin-2-yl-methoxy)phenyl]-2-cyclopentyl-essigsäure und 5 g (22 mmol) Bromessigsäurebenzylester analog der Vorschrift von Beispiel 13.
Ausbeute: 8,03 g (79% der Theorie)
Festpunkt: 63 - 65°C (Hydrochlorid)

### Beispie 15

### 2-[4-(Chinolin-2-yl-methoxy)phenyl]-2-cyclopentyl-essigsäuremethyloxycarbonylmethylamid

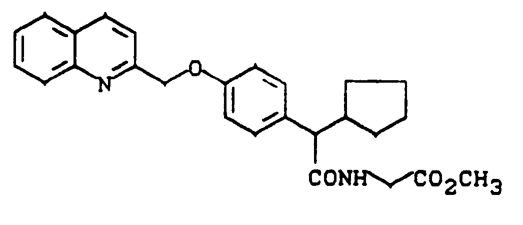

7,22 g (20 mmol) 2-[4-(Chinolin-2-yl-methoxy)phenyl]-2-cyclopentylessigsäure und 3,0 g (24 mmol) Glycinmethylester-Hydrochlorid werden in 75 ml Dimethylformamid gelöst. Nach Abkühlen auf 0°C werden 6,6 g (24 mmol) Phosphorsäurediphenylesterazid, gelöst in 25 ml Dimethylformamid, zugetropft und 30 min nachgerührt. Anschließend werden 7,3 g (72 mmol) Triethylamin zugetropft und 4 h bei 0°C und 15 h bei 25°C nachgerührt. Die Reaktionslösung wird auf 300 g Eis gegossen und 3 mal mit Essigester extrahiert. Die organischen Phasen werden 1 mal mit 1 normaler Salzsäure, 1 mal mit Wasser gewaschen, über Natriumsulfat getrocknet und eingeengt. Das Produkt wird aus Methanol unkristallisiert.
Ausbeute: 5,34 g (62% der Theorie)
Festpunkt: 134 - 136°C

### Beispiel 16

### 2-[4-(Chinolin-2-yl-methoxy)phenyl]-2-(1-dekalinyl)-essigsäuremethylester

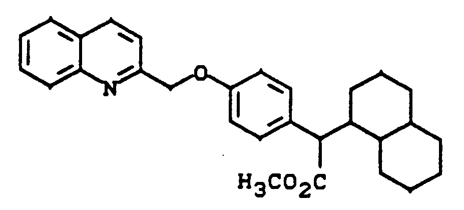

Die Darstellung erfolgt aus 15,4 g (50 mmol) 4-(Chinolin-2-yl-methoxy)phenyl-essigsäuremethylester und 9,55 g (55 mmol) 1-Chlor-dekalin analog der Vorschrift von Beispiel 2.
Ausbeute: 3,11 g (14% der Theorie)
Festpunkt: 118°C

### Beispiel 17

### 2-[4-(Chinolin-2-yl-methoxy)phenyl]-2-cyclopentyl-essigsäure-tert.butyloxycarbonylmethylester

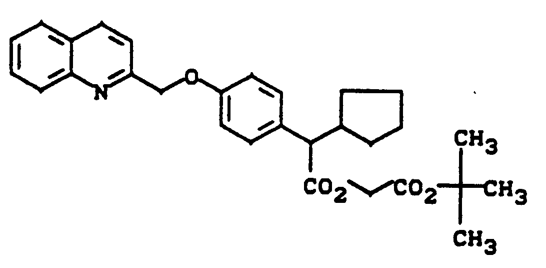

Die Darstellung erfolgt aus 3 g (8,3 mmol) 2-[4-(Chinolin-2-yl-methoxy)phenyl]-2-cyclopentyl-essigsäure und 1,77 g (9,1 mmol) Bromessigsäure-tert.butylester analog der Vorschrift von Beispiel 13.
Ausbeute: 3,18 g (80,5% der Theorie)
Festpunkt: 88 - 91°C

### Beispiel 18

### 2-[4-(Chinolin-2-yl-methoxy)phenyl]-2-cyclopentyl-essigsäure-pivaloyloxymethylester

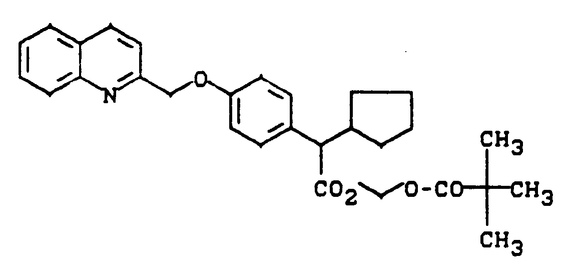

Die Darstellung erfolgt aus 3 g (8,3 mmol) 2-[4-(Chinolin-2-yl-methoxy)phenyl]-2-cyclopentyl-essigsäure und 1,37 g (9,1 mmol) Pivalinsäurechlormethylester analog der Vorschrift von Beispiel 13.
Ausbeute: 1,38 g (35% der Theorie)
Festpunkt: 30 - 32°C

### Beispiel 19

### 2-[4-(Chinolin-2-yl-methoxy)phenyl]-2-cyclopenyl-essigsäuremethoxycarbonylmethylester

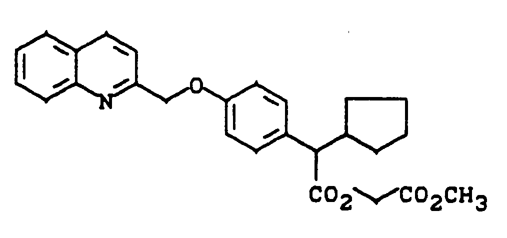

Die Darstellung erfolgt aus 3 g (8,3 mmol) 2-[4-(Chinolin-2-yl-methoxy)phenyl]-2-cyclopentyl-essigsäure und 1,39 g (9,1 mmol) Bromessigsäuremethylester analog der Vorschrift von Beispiel 13.
Ausbeute: 3,37 g (94% der Theorie)
Festpunkt: 90 - 93°C

### Beispiel 20

### 2-[4-(Chinolin-2-yl-methoxy)phenyl]-2-(1-dekalinyl)-essigsäure

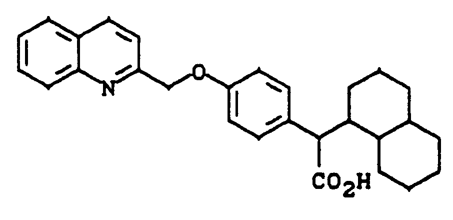

Die Darstellung erfolgt aus 610 mg (1,37 mmol) 2-[4-(Chinolin-2-yl-methoxy)phenyl]-2-(1-dekalinyl)-essigsäuremethylester analog der Vorschrift von Beispiel 7.
Ausbeute: 470 mg (80% der Theorie)
Festpunkt: 200 - 207°C

### Beispiel 21

### 2-[4-(Chinolin-2-yl-methoxy)phenyl]-2-cyclopentyl-essigsäurecarboxymethylamid

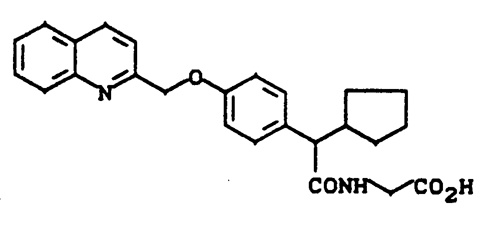

Die Darstellung erfolgt aus 3 g (69 mmol) 2-[4-(Chinolin-2-yl-methoxy)phenyl]-2-cyclopentyl-essigsäuremethyloxycarbonyl-methylamid analog der Vorschrift von Beispiel 7.
Ausbeute: 2,47 g (85% der Theorie)
Festpunkt: 182 - 185°C

### Beispiel 22

### 2-[4-(Chinolin-2-yl-methoxy)phenyl]-2-cyclopentyl-essigsäure-Natriumsalz

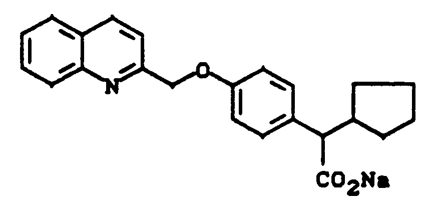

10 g (27,7 mmol) 2-[4-(Chinolin-2-yl-methoxy)phenyl]-2-cyclopentylessigsäure werden in 100 ml Wasser gelöst. Nach Zugabe von 27,7 ml 1-normaler Natronlauge wird 1 h bei 25°C gerührt, danach eingeengt und bei 100°C im Vakuum getrocknet.
Ausbeute: quantitativ
Festpunkt: > 230°C

### Beispiel 23

### 2-[4-(Chinolin-2-yl-methoxy)phenyl]-3-cyclopentyl-propionsäuremethylester

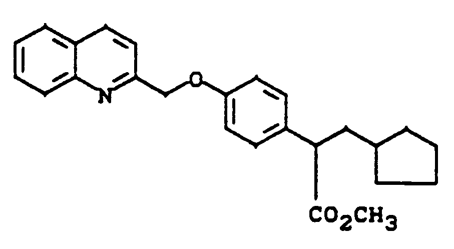

Die Darstellung erfolgt aus 6,2 g (20 mmol) 4-(Chinolin-2-yl-methoxy)phenylessigsäuremethylester und 3,3 g (20 mmol) Brommethyl-cyclopentan analog der Vorschrift von Beispiel 2.
Ausbeute: 5,1 g (65,5 % der Theorie)
Festpunkt: 66 - 68°C

### Beispiel 24

### 2-[4-(Chinolin-2-yl-methoxy)phenyl]-3-cyclopentyl-propionsäure

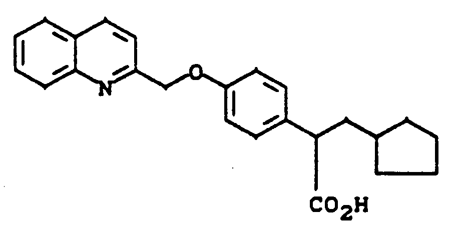

Die Darstellung erfolgt aus 5 g (12,8 mmol) 2-[4-(Chinolin-2-yl-methoxy)phenyl]-3-cyclopentyl-propionsäuremethylester analog der Vorschrift von Beispiel 7.
Ausbeute: 2,5 g (52 % der Theorie)
Festpunkt: 126 - 128°C

### Beispiel 25

### 2-[4-(Chinolin-2-yl-methoxy)phenyl]-2-(cyclohex-2-enyl)essigsäure

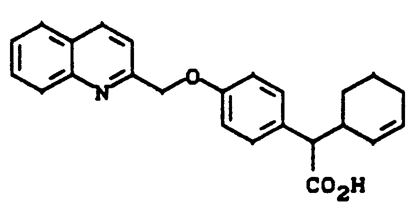

Die Darstellung erfolgt aus 24,34 g (62,8 mmol) 2-[4-(Chinolin-2-yl-methoxy)phenyl]-2-(cyclohex-2-enyl)essigsäuremethylester analog der Vorschrift von Beispiel 7.
Ausbeute: 18,3 g (78 % der Theorie)
Festpunkt: 188 - 192°C

### Beispiel 26

### 2-[4-(Chinolin-2-yl-methoxy)phenyl]-3-cyclopentyl-essigsäurecarboxymethylester

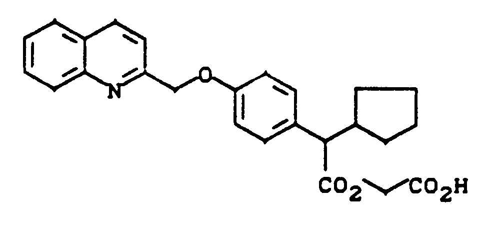

6,91 g (13,5 mmol) 2-[4-(Chinolin-2-yl-methoxy)phenyl]-2-cyclopentyl-essigsäurebenzyloxycarbonylmethylester werden in 100 ml Essigester und 10 ml Triethylamin gelöst, 0,5 g Palladium-Katalysator (10%ig auf Kohle) zugegeben und bei Normaldruck bei 25°C hydriert. Nach Aufnahme der theoretischen Menge Wasserstoff wird der Katalysator abfiltriert. Nach Einengen im Vakuum wird der Rückstand aus Methanol umkristallisiert.
Ausbeute: 3,15 g (55,6 % der Theorie)
Festpunkt: 168 - 171°C

### Beispiel 27

### 2-[4-(6-Fluorchinolin-2-yl-methoxy)phenyl]-2-cyclopentylessigsäuremethylester

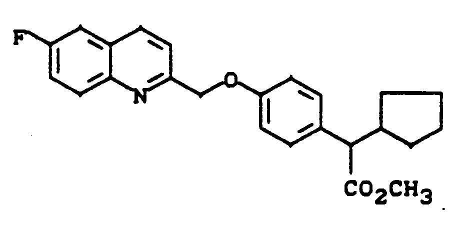

4,68 g (20,4 mmol) 2-[4-(Hydroxyphenyl)-2-cyclopentyl-essigsäuremethylester werden in 50 ml Dimethylformamid gelöst. Nach Zugabe von 2,82 g (20,4 mmol) Kaliumcarbonat wird 1 h bei 50°C gerührt, 4 g (20,4 mmol) 2-Chlormethyl-6-fluor-chinolin zugegeben und weitere 15 h bei 50°C gerührt. Nach Einengen im Vakuum wird der Rückstand zwischen Wasser und Essigester verteilt, die organische Phase über Natriumsulfat getrocknet, eingeengt und der Rückstand aus Methanol umkristallisiert.
Ausbeute: 7,36 g (91,6 % der Theorie)
Festpunkt: 117 - 119°C

### Beispiel 28

### 2-[4-(6-Fluorchinolin-2-yl-methoxy)phenyl]-2-cyclopentylessigsäure

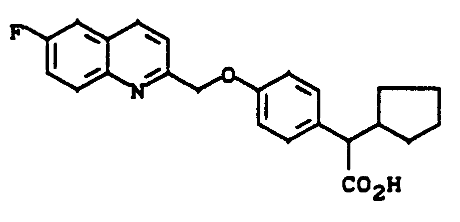

Die Darstellung erfolgt aus 7 g (17,8 mmol) 2-[4-(Fluorchinolin-2-yl-methoxy)-phenyl]-2-cyclopentyl-essigsäuremethylester analog der Vorschrift von Beispiel 7.
Ausbeute: 4,51 g (67 % der Theorie)
Festpunkt: 182 - 184°C

### Beispiel 29

### 2-[4-(Chinolin-2-yl-methoxy)phenyl]-2-norbornyl-essigsäuremethylester

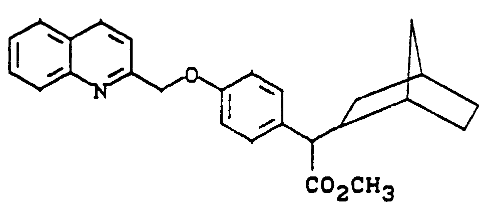

Die Darstellung erfolgt aus 6,2 g (20 mmol) 2-[4-(Chinolin-2-yl-methoxy)phenyl]essigsäuremethylester und 3,5 g (20 mmol) exo-2-Norbornylchlorid analog der Vorschrift von Beispiel 2. Zur Reinigung wird an Kieselgel 60 (Eluens: Toluol/Essigsäure 9 : 1) chromatographiert.
Ausbeute: 0,2 g (2,5 % der Theorie)
Festpunkt: 123 - 125°C

### Beispiel 30

### 2-[4-(Chinolin-2-yl-methoxy)-phenyl]-2-norbornyl-essigsäure

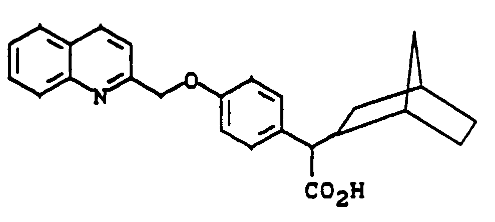

Die Darstellung erfolgt aus 0,4 g (1 mmol) 2-[4-(Chinolin-2-yl-methoxy)phenyl]-2-norbornyl-essigsäuremethylester analog der Vorschrift von Beispiel 7.
Ausbeute: 0,36 g (93 % der Theorie)
Festpunkt: 158 - 160°C

### Beispiel 31

### 4-Benzyloxyphenylessigsäuremethylester

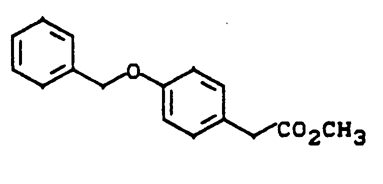

397 g 4-Hydroxyphenylessigsäuremethylester und 330 g Kaliumcarbonat werden in 2 l Dimethylformamid 1 h bei 25°C gerührt. Danach werden 302 g Benzylchlorid zugegeben und 15 h auf 50°C erwärmt. Nach Einengen im Vakuum wird der Rückstand zwischen Wasser und Essigester verteilt, die organische Phase über Natriumsulfat getrocknet und eingeengt. Das Produkt wird aus Methanol umkristallisiert.
Ausbeute: 511 g (83 % der Theorie)
Festpunkt: 60°C

### Beispiel 32

### 2-(4-Benzyloxyphenyl)-2-cyclopentylessigsäuremethylester

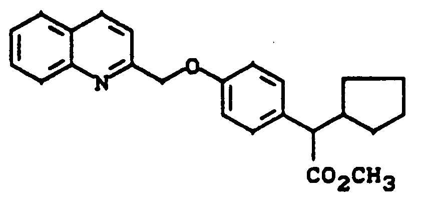

256,3 g (1 mol) 4-Benzyloxyphenylessigsäuremethylester werden in 1 l Dimethylformamid gelöst und unter Schutzgas (Argon) bei 0°C in eine Suspension von 24 g (1 mol) Natriumhydrid in 100 ml Dimethylformamid getropft. Nach beendeter H₂-Entwicklung wird 2 h bei 0°C nachgerührt Anschließend werden bei gleicher Temperatur 149 g (1 mol) Cyclopentylbromid, in 400 ml Dimethylformamid gelöst, zugetropft. Nach beendeter Zugabe wird 15 h bei Raumtemperatur nachgerührt. Das Lösungsmittel wird im Vakuum eingeengt und der Rückstand mit heißem Wasser (80°C) versetzt. Unter Rühren (KPG-Rührer) wird langsam abgekühlt. Das kristallisierte Produkt wird abgesaugt, gut mit Wasser gewaschen, getrocknet und aus Methanol unkristallisiert. Ausbeute: 276 g (85 % der Theorie)
Festpunkt: 77 - 78°C (Methanol)

### Beispiel 33

### 2-Cyclopentyl-2-(4-hydroxyphenyl)

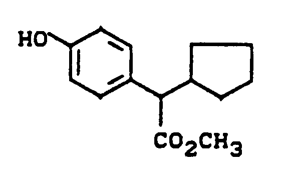

65 g (0,2 mol) 2-(4-Benzyloxyphenyl)-2-cyclopentylessigsäuremethylester werden in 100 ml Tetrahydrofuran, 200 ml Ethanol und 100 ml Triethylamin gelöst. Nach Zugabe von 1,5 g Palladium-Katalysator (10%ig auf Kohle) wird 2 h bei 3 bar Wasserstoff hydriert. Der Katalysator wird abfiltriert, das Filtrat eingeengt und an Kieselgel (Eluens: Methylenchlorid) chromatographiert. Man erhält ein zähes Öl.
Ausbeute: 43,7 g (93 % der Theorie)

### Beispiele 34 A und 34 B

### Diastereomere von 2-[4-Chinolin-2-yl-methoxy)phenyl]-2-cyclopentylessigsäure[(L)-2-hydroxy-1-phenylethyl]amid

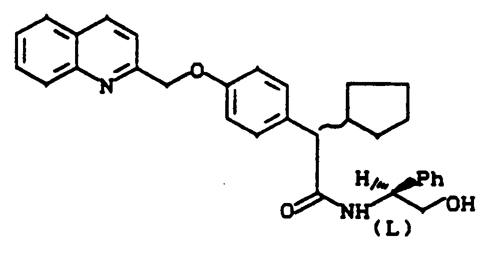

7,2 g (20 mmol) 2-[4-Chinolin-2-yl-methoxy)phenyl]-2-cyclopentylessigsäure und 3,3 g (24 mmol) (L)-Phenylglycinol werden in 100 ml Dimethylformamid gelöst. Zu der auf -10°C gekühlten Lösung werden 6,6 g (24 mmol) Phosphorsäurediphenylesterazid in 25 ml Dimethylformamid langsam zugetropft, anschließend 4,8 g (48 mmol) Triethylamin zugegeben und 15 h bei -10°C gerührt. Das Reaktionsgemisch wird auf Eis gegeben, das Rohprodukt abfiltriert, mit Wasser gewaschen und getrocknet. Dreimalige Umkristallisation aus Ethanol ergibt Diastereomer 34 A. Diastereomer 34 B wird durch dreimalige Umkristallisation der vereinigten Mutterlaugen aus Dichlormethan erhalten.
- Beispiele 34 A:: Ausbeute: 1,93 g (20,1 % der Theorie)
Festpunkt: 201 - 203°C (EtOH)
- Beispiel 34 B:: Ausbeute: 1,52 g (15,8 % der Theorie)
Festpunkt: 158 - 159°C (CH₂Cl₂)

### Beispiel 35

### (+)-4-(2-Chinolin-2-yl-methoxy)phenyl]-2-cyclopentyl-essigsäure

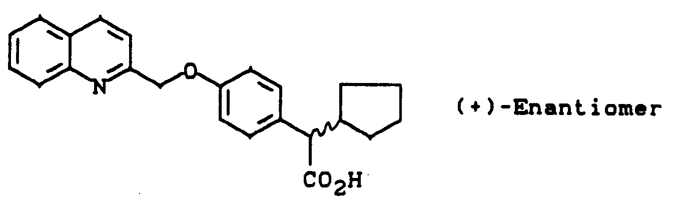

4,8 g (10 mmol) Diastereomer A aus Beispiel 34 werden in 50 ml Dioxan und 50 ml 5 normaler Schwefelsäure 24 h unter Rückfluß erwärmt. Nach Abkühlen auf 0°C wird mit 5 normaler Natronlauge pH 3 eingestellt. Das Produkt wird abgesaugt und aus Ethanol umkristallisiert.
Ausbeute: 2,38 g (65,8 % der Theorie)
α_{D}²⁵ = +40,9 (c = 1, CHCl₃)
Schmelzpunkt: 170-172°C

### Beispiel 36

### (-)-4-[2-Chinolin-2-yl-methoxy)-phenyl]-2-cyclopentyl-essigsäure

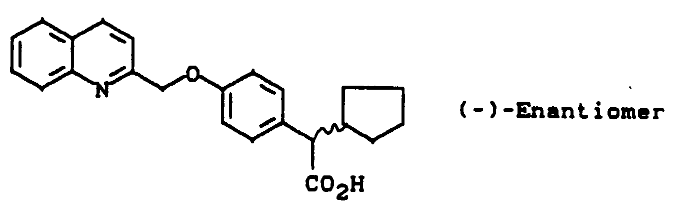

Die Darstellung der Verbindung 36 erfolgt analog der Vorschrift von Beispiel 35 unter Einsatz von 4,8 g (10 mmol) des Diastereomeren B aus Beispiel 34.
Ausbeute: 2,28 g (63,2 % der Theorie)
α_{D}²⁵ = - 40,7 (C = 1, CHCl₃)
Schmelzpunkt: 170-172°C

### Beispiel 37 (Anwendungsbeispiel)

Als Maß für eine Lipoxygenase-Hemmung wurde die Freisetzung von Leukotrien B₄ (LTB₄) aus polymorphkernigen Rattenleukozyten (PMN) nach Zugabe von Substanzen und Ca-Ionophor mittels reverse phase HPLC nach Borgeat, P. et al, Proc. Nat, Acad. Sci. (USA) 76, 2148-2152 (1979) bestimmt.

In der Tabelle 1 sind beispielhaft die nach diesem Test erzielten Werte einiger erfindungsgemäßer Verbindungen aufgeführt:

**Tabelle 1**

| Lipoxygenasehemmung | |
|---|---|
| Bsp.-Nr. | LO-Hemmung IC₅₀ (µM) |
| 7 | 0,14 |
| 8 | 0,01 |
| 9 | 0,04 |

## Patentansprüche (Patentansprüche für folgende(n) Vertragsstaat(en): AT, BE, CH, DE, FR, GB, GR, IT, LI, NL, SE)

1. Substituierte 4-(Chinolin-2-yl-methoxy)phenyl-essigsäure-Derivate der Formel 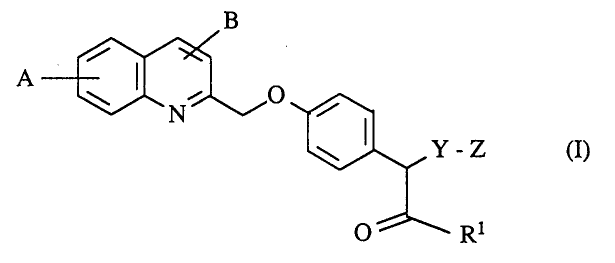 worin
R¹ - für eine Gruppe der Formel
-OR² oder 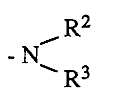 steht, wobei
R² und R³ gleich oder verschieden sind und
- für Wasserstoff, C₁-C₆-Alkyl Benzyl, Phenyl oder eine Gruppe der Formel 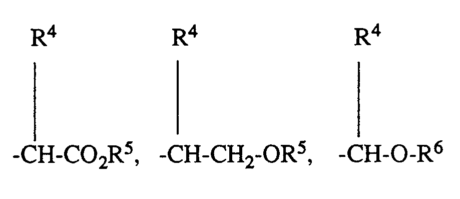 oder 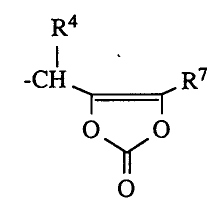 stehen, wobei
R⁴ - für Wasserstoff, C₁-C₆-Alkyl, Phenyl oder Benzyl steht, das gegebenenfalls substituiert sein kann durch Hydroxy, C₁-C₆-Alkoxycarbonyl, Carboxyl, C₁-C₆-Alkylthio, Thienyl, Furyl, Pyridyl, Pyrimidyl, Pyrazinyl, Pyridazinyl, Chinolyl, Isochinolyl, Chinazolyl, Chinoxalyl, Thiazolyl, Benzothiazolyl, Isothiazolyl, Oxazolyl, Benzoxazolyl, Isoxazolyl, Imidazolyl, Benzimidazolyl, Pyrazolyl, Indolyl und Isoindolyl oder Carbymoyl,
R⁵ - für Wasserstoff, C₁-C₆-Alkyl, Phenyl oder Benzyl steht,
R⁶ - für eine Gruppe der Formel -COR⁵ oder -CO₂R⁵ steht, wobei R⁵ die oben angegebene Bedeutung hat,
und
R⁷ - für Wasserstoff, C₁-C₆-Alkyl oder Phenyl steht,
Y - für eine Gruppe der Formel 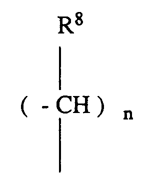 steht, wobei
R⁸ - für Wasserstoff, C₁-C₆-Alkyl oder Phenyl steht,
und
n - eine Zahl von 0 bis 5 bedeutet,
Z - für Norbornyl steht, oder
für eine Gruppe der Formel 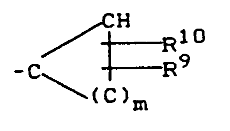 oder 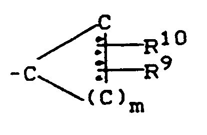 steht, wobei
R⁹ und R¹⁰ gleich oder verschieden sind und Wasserstoff, C₁-C₆-Alkyl oder Phenyl bedeuten, oder
R⁹ und R¹⁰ gemeinsam einen gesättigten carbocyclischen Ring mit bis zu 6 Kohlenstoffatomen bilden können und
m eine Zahl von 1 bis 6 bedeutet,
und
A und B gleich oder verschieden sind und Wasserstoff, Methyl, Ethyl, Fluor, Chlor oder Brom bedeuten,
sowie deren Salze.

2. Substituierte 4-(Chinolin-2-yl-methoxy)phenylessigsäure-Derivate der Formel (I) nach Anspruch 1, worin
R¹ - für eine Gruppe der Formel
-OR² oder 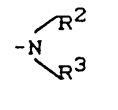 steht, wobei
R² und R³ gleich oder verschieden sind und
- für Wasserstoff, Methyl, Ethyl, Propyl, Isopropyl, Butyl, tert.Butyl, Phenyl oder Benzyl stehen, oder für eine Gruppe der Formel 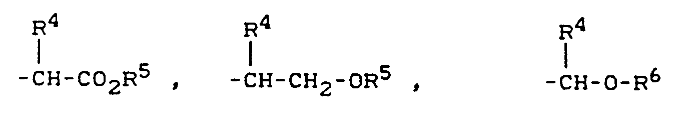 oder 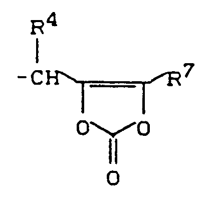 stehen, wobei
R⁴ - für Wasserstoff, Methyl, Ethyl, Propyl, Isopropyl, Butyl, tert.-Butyl, Benzyl oder Phenyl steht, das gegebenenfalls substituiert sein kann durch Hydroxy, Methoxycarbonyl, Ethoxycarbonyl, Propoxycarbonyl, Carboxyl, Methylthio, Ethylthio, Propylthio, Imidazolyl oder Carbamoyl
R⁵ - für Wasserstoff, Methyl, Ethyl, Propyl, Isopropyl, Butyl, tert.-Butyl, Phenyl oder Benzyl steht,
und
R⁶ - für eine Gruppe der Formel -COR⁵ oder -CO₂R⁵ steht, wobei R⁵ die oben angegebene Bedeutung hat
und
R⁷ - für Wasserstoff, Methyl, Ethyl, Propyl, Isopropyl, Butyl, tert.Butyl oder Phenyl steht,
Y - für eine Gruppe der Formel 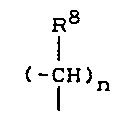 steht, wobei
R⁸ - für Wasserstoff, Methyl, Ethyl, Propyl, Isopropyl, Butyl, tert.Butyl oder Phenyl steht,
und
n - eine Zahl von 0 bis 5 bedeutet,
Z - für Norbornyl steht oder
für eine Gruppe der Formel 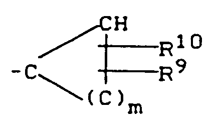 oder 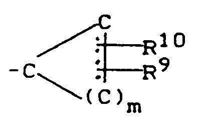 steht, wobei
R⁹ und R¹⁰ gleich oder verschieden sind und Wasserstoff, Methyl, Ethyl, n-Propyl, iso-Propyl, Butyl oder tert.-Butyl bedeuten, oder
R⁹ und R¹⁰ gemeinsam einen gesättigten carbocyclischen Ring mit bis zu 6 Kohlenstoffatomen bilden können und
m - eine Zahl von 1 bis 6 bedeutet,
und
A und B gleich oder verschieden sind und Wasserstoff, Methyl, Ethyl, Fluor oder Chlor bedeuten,
sowie deren Salze.

3. Substituierte 4-(Chinolin-2-yl-methoxy)phenyl-essigsäure-Derivate nach Anspruch 1, wobei
R¹ - für eine Gruppe der Formel
-OR² oder 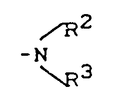 steht, wobei
R² und R³ gleich oder verschieden sind und
- für Wasserstoff oder Methyl stehen, oder
- für eine Gruppe der Formel 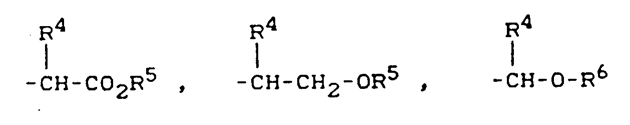 oder 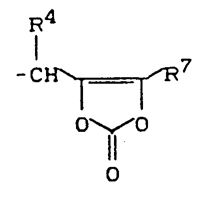 stehen, wobei
R⁴ - für Wasserstoff, Methyl oder Phenyl steht,
R⁵ - für Wasserstoff, Methyl, Ethyl, tert.-Butyl oder Benzyl steht,
R⁶ - für eine Gruppe der Formel -COR⁵ steht, wobei R⁵ die oben angegebene Bedeutung hat, und
R⁷ - für Methyl steht,
Y - für eine Gruppe der Formel 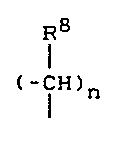 steht, wobei
R⁸ - für Wasserstoff oder Methyl steht,
und
n - eine Zahl 0 oder 1 bedeutet,
Z - für Norbornyl steht, oder
für eine Gruppe der Formel 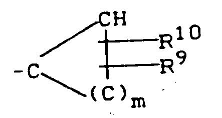 oder 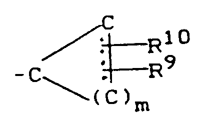 steht, wobei
R⁹ und R¹⁰ gleich oder verschieden sind und Wasserstoff oder Methyl bedeuten, oder
R⁹ und R¹⁰ gemeinsam einen Cyclohexylring bilden, und
m - eine Zahl 1, 2, 3, 4 oder 5 bedeutet,
A und B Wasserstoff oder Fluor bedeuten,
sowie deren Salze.

4. (+)-4-[(2-Chinolin-2-yl-methoxy)phenyl]-2-cyclopentyl-essigsäure, gemäß Anspruch 1.

5. (-)-4-[(2-Chinolin-2-yl-methoxy)phenyl]-2-cyclopentyl-essigsäure, gemäß Anspruch 1.

6. Verfahren zur Herstellung von substituierten 4-(Chinolin-2-yl-methoxy)phenyl-essigsäure-Derivate nach Anspruch 1, dadurch gekennzeichnet, daß man
[A] 4-(Chinolin-2-yl-methoxy)phenylessigsäureester der allgemeinen Formel (Ia) 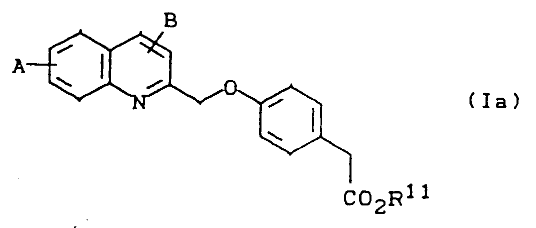 in welcher
R¹¹ - für Alkyl steht und
A und B die oben angegebene Bedeutung haben,
mit Verbindungen der allgemeinen Formel (II)
Y-Z-X (II)
in welcher
Y und Z die oben angegebene Bedeutung haben, und
X - für Chlor, Brom oder Iod steht,
alkyliert und im Falle der Säuren die Ester verseift,
oder indem man
[B] die Säuren der allgemeinen Formel (Ib) 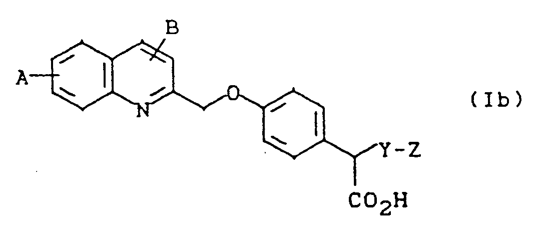 in welcher
A, B, Y und Z die oben angegebene Bedeutung haben,
mit Verbindungen der allgemeinen Formel (III)
X-R¹² (III)
in welcher
R¹² - für eine Gruppe der Formel 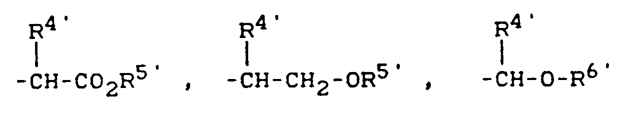 oder 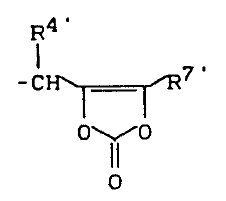 steht, wobei
R⁴' - für Alkyl, Aralkyl oder Aryl steht, das gegebenenfalls substituiert sein kann durch Hydroxy, Carboxyl, Alkoxycarbonyl, Alkylthio, Heteroaryl oder Aminocarbonyl,
R⁵' - für Alkyl, Aryl oder Aralkyl steht,
R⁶' - für eine Gruppe der Formel -COR⁵' oder -CO₂R⁵' steht, wobei
R⁵' die oben angegebene Bedeutung hat,
R⁷' - für Alkyl oder Aryl steht,
und
X die oben angegebene Bedeutung hat,
verestert und im Fall der Säuren die Ester einer hydrogenolytischen Spaltung unterzieht,
oder indem man
[C] die Säuren der allgemeinen Formel (Ib) mit Aminen der allgemeinen Formel (IV) 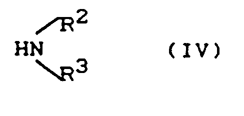 in welcher
R² und R³ die oben angegebene Bedeutung haben,
mit der Maßgabe, daß R⁵ nicht Wasserstoff bedeutet, wenn R² oder R³ für die Gruppe 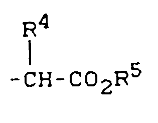 steht wobei
R⁴ und R⁵ die obengenannte Bedeutung haben,
in Gegenwart von üblichen Aktivierungsreagentien amidiert und im Fall der Säuren die Ester verseift
oder indem man
[D] Phenole der allgemeinen Formel (V) 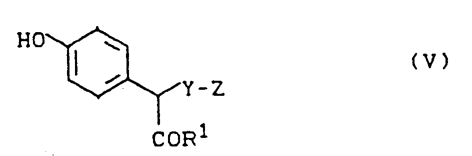 in welcher
R¹, Y und Z die oben angegebene Bedeutung haben
mit
2-Halogenmethylchinolinen der Formel (VI) 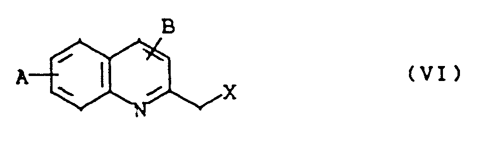 in welcher
A, B und X die oben angegebene Bedeutung haben,
verethert und im Fall der Säuren die Ester verseift.

7. Verfahren nach Anspruch 6, dadurch gekennzeichnet, daß es im Temperaturbereich von 0 bis 150°C durchgeführt wird.

8. Arzneimittel, enthaltend substituierte 4-(Chinolin-2-yl-methoxy)phenyl-essigsäure-Derivate nach den Ansprüchen 1 bis 5.

9. Arzneimittel nach Anspruch 8, enthaltend 0,5 bis 90 Gew.-% an substituierten 4-(Chinolin-2-yl-methoxy)phenyl-essigsäure-Derivate.

10. Verwendung von substituierten 4-(Chinolin-2-yl-methoxy)phenyl-essigsäure-Derivate nach den Ansprüchen 1 bis 5 zur Herstellung von Arzneimitteln.

## Patentansprüche (Patentansprüche für folgende(n) Vertragsstaat(en): ES)

1. Verfahren zur Herstellung von substituierten 4-(Chinolin-2-yl-methoxy)phenyl-essigsäure-Derivaten der Formel 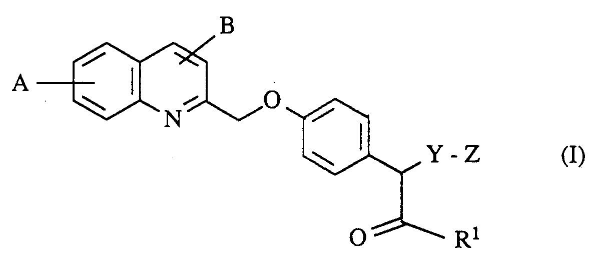 worin
R¹ - für eine Gruppe der Formel
-OR² oder 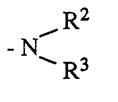 steht, wobei
R² und R³ gleich oder verschieden sind und
- für Wasserstoff, C₁-C₆-Alkyl Benzyl, Phenyl oder eine Gruppe der Formel 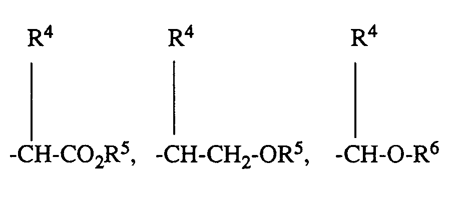 oder 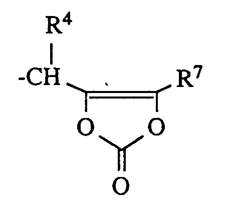 stehen, wobei
R⁴ - für Wasserstoff, C₁-C₆-Alkyl, Phenyl oder Benzyl steht, das gegebenenfalls substituiert sein kann durch Hydroxy, C₁-C₆-Alkoxycarbonyl, Carboxyl, C₁-C₆-Alkylthio, Thienyl, Furyl, Pyridyl, Pyrimidyl, Pyrazinyl, Pyridazinyl, Chinolyl, Isochinolyl, Chinazolyl, Chinoxalyl, Thiazolyl, Benzothiazolyl, Isothiazolyl, Oxazolyl, Benzoxazolyl, Isoxazolyl, Imidazolyl, Benzimidazoly, Pyrazolyl, Indolyl und Isoindolyl oder Carbymoyl,
R⁵ - für Wasserstoff, C₁-C₆-Alkyl, Phenyl oder Benzyl steht,
R⁶ - für eine Gruppe der Formel -COR⁵ oder -CO₂R⁵ steht, wobei R⁵ die oben angegebene Bedeutung hat,
und
R⁷ - für Wasserstoff, C₁-C₆-Alkyl oder Phenyl steht,
Y - für eine Gruppe der Formel 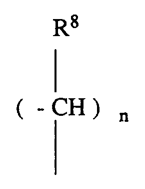 steht, wobei
R⁸ - für Wasserstoff, C₁-C₆-Alkyl oder Phenyl steht,
und
n - eine Zahl von 0 bis 5 bedeutet,
Z - für Norbornyl steht, oder
für eine Gruppe der Formel 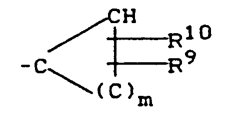 oder 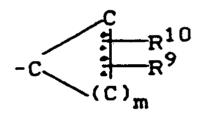 steht, wobei
R⁹ und R¹⁰ gleich oder verschieden sind und Wasserstoff, C₁-C₆-Alkyl oder Phenyl bedeuten, oder
R⁹ und R¹⁰ gemeinsam einen gesättigten carbocyclischen Ring mit bis zu 6 Kohlenstoffatomen bilden können und
m eine Zahl von 1 bis 6 bedeutet,
und
A und B gleich oder verschieden sind und Wasserstoff, Methyl, Ethyl, Fluor, Chlor oder Brom bedeuten,
sowie deren Salze, dadurch gekennzeichnet, daß man
[A] 4-(Chinolin-2-yl-methoxy)phenylessigsäureester der allgemeinen Formel (Ia) 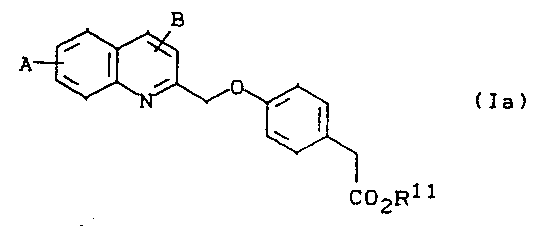 in welcher
R¹¹ - für Alkyl steht und
A und B die oben angegebene Bedeutung haben,
mit Verbindungen der allgemeinen Formel (II)
Y-Z-X (II)
in welcher
Y und Z die oben angegebene Bedeutung haben, und
X - für Chlor, Brom oder Iod steht,
alkyliert und im Falle der Säuren die Ester verseift,
oder indem man
[B] die Säuren der allgemeinen Formel (Ib) 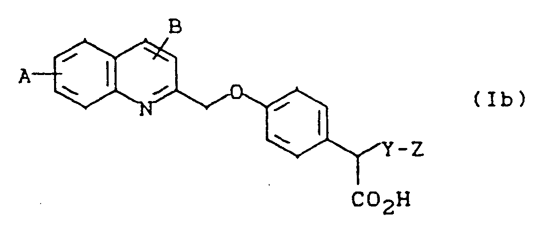 in welcher
A, B, Y und Z die oben angegebene Bedeutung haben,
mit Verbindungen der allgemeinen Formel (III)
X-R¹² (III)
in welcher
R¹² - für eine Gruppe der Formel 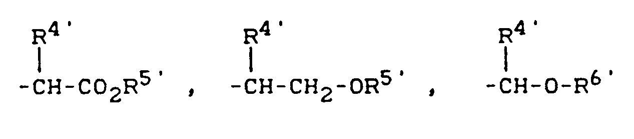 oder 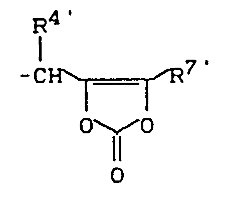 steht, wobei
R⁴' - für Alkyl, Aralkyl oder Aryl steht, das gegebenenfalls substituiert sein kann durch Hydroxy, Carboxyl, Alkoxycarbonyl, Alkylthio, Heteroaryl oder Aminocarbonyl,
R⁵' - für Alkyl, Aryl oder Aralkyl steht,
R⁶' - für eine Gruppe der Formel -COR⁵' oder -CO₂R⁵' steht, wobei
R⁵' die oben angegebene Bedeutung hat,
R⁷' - für Alkyl oder Aryl steht,
und
X die oben angegebene Bedeutung hat,
verestert und im Fall der Säuren die Ester einer hydrogenolytischen Spaltung unterzieht,
oder indem man
[C] die Säuren der allgemeinen Formel (Ib) mit Aminen der allgemeinen Formel (IV) 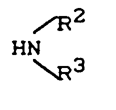 in welcher
R² und R³ die oben angegebene Bedeutung haben,
mit der Maßgabe, daß R⁵ nicht Wasserstoff bedeutet, wenn R² oder R³ für die Gruppe 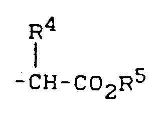 steht wobei
R⁴ und R⁵ die obengenannte Bedeutung haben,
in Gegenwart von üblichen Aktivierungsreagentien amidiert und im Fall der Säuren die Ester verseift
oder indem man
[D] Phenole der allgemeinen Formel (V) 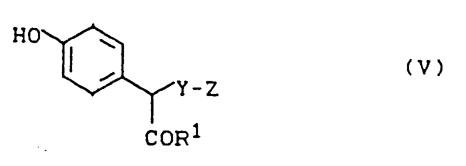 in welcher
R¹, Y und Z die oben angegebene Bedeutung haben
mit
2-Halogenmethylchinolinen der Formel (VI) 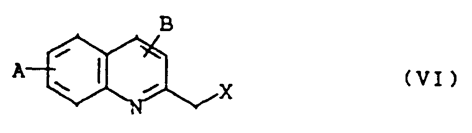 in welcher
A, B und X die oben angegebene Bedeutung haben,
verethert und im Fall der Säuren die Ester verseift.

2. Verfahren nach Anspruch 1 zur Herstellung von substituierten 4-(Chinolin-2-yl-methoxy)phenyl-essigsäure-Derivaten der Formel nach Anspruch 1, wobei
R¹ - für eine Gruppe der Formel
-OR² oder 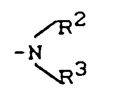 steht, wobei
R² und R³ gleich oder verschieden sind und
- für Wasserstoff, Methyl, Ethyl, Propyl, Isopropyl, Butyl, tert.Butyl, Phenyl oder Benzyl stehen, oder für eine Gruppe der Formel 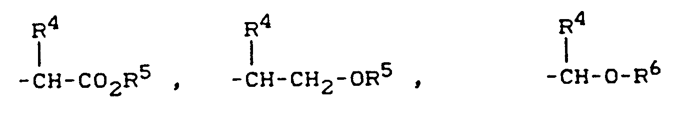 oder 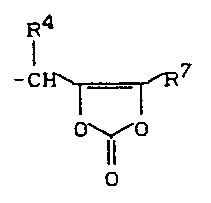 stehen, wobei
R⁴ - für Wasserstoff, Methyl, Ethyl, Propyl, Isopropyl, Butyl, tert.-Butyl, Benzyl oder Phenyl steht, das gegebenenfalls substituiert sein kann durch Hydroxy, Methoxycarbonyl, Ethoxycarbonyl, Propoxycarbonyl, Carboxyl, Methylthio, Ethylthio, Propylthio, Imidazolyl oder Carbamoyl
R⁵ - für Wasserstoff, Methyl, Ethyl, Propyl, Isopropyl, Butyl, tert.-Butyl, Phenyl oder Benzyl steht,
und
R⁶ - für eine Gruppe der Formel -COR⁵ oder -CO₂R⁵ steht, wobei R⁵ die oben angegebene Bedeutung hat
und
R⁷ - für Wasserstoff, Methyl, Ethyl, Propyl, Isopropyl, Butyl, tert. Butyl oder Phenyl steht,
Y - für eine Gruppe der Formel 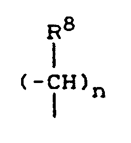 steht, wobei
R⁸ - für Wasserstoff, Methyl, Ethyl, Propyl, Isopropyl, Butyl, tert.Butyl oder Phenyl steht,
und
n - eine Zahl von 0 bis 5 bedeutet,
Z - für Norbornyl steht oder
für eine Gruppe der Formel 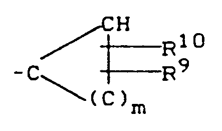 oder 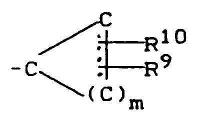 steht, wobei
R⁹ und R¹⁰ gleich oder verschieden sind und Wasserstoff, Methyl, Ethyl, n-Propyl, iso-Propyl, Butyl oder tert.-Butyl bedeuten, oder
R⁹ und R¹⁰ gemeinsam einen gesättigten carbocyclischen Ring mit bis zu 6 Kohlenstoffatomen bilden können und
m - eine Zahl von 1 bis 6 bedeutet,
und
A und B gleich oder verschieden sind und Wasserstoff, Methyl, Ethyl, Fluor oder Chlor bedeuten,
sowie deren Salze.

3. Verfahren nach Anspruch 1 zur Herstellung von substituierten 4-(Chinolin-2-yl-methoxy)phenyl-essigsäure-Derivaten der Formel nach Anspruch 1, wobei
R¹ - für eine Gruppe der Formel
-OR² oder 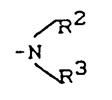 steht, wobei
R² und R³ gleich oder verschieden sind und
- für Wasserstoff oder Methyl stehen, oder
- für eine Gruppe der Formel 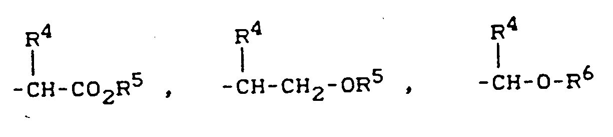 oder 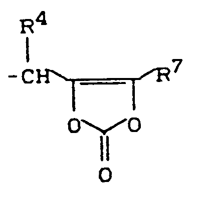 stehen, wobei
R⁴ - für Wasserstoff, Methyl oder Phenyl steht,
R⁵ - für Wasserstoff, Methyl, Ethyl, tert.-Butyl oder Benzyl steht,
R⁶ - für eine Gruppe der Formel -COR⁵ steht, wobei R⁵ die oben angegebene Bedeutung hat, und
R⁷ - für Methyl steht,
Y - für eine Gruppe der Formel 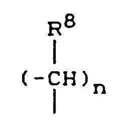 steht, wobei
R⁸ - für Wasserstoff oder Methyl steht,
und
n - eine Zahl 0 oder 1 bedeutet,
Z - für Norbornyl steht, oder
für eine Gruppe der Formel 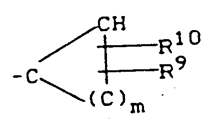 oder 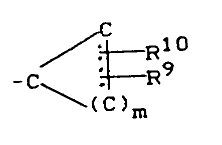 steht, wobei
R⁹ und R¹⁰ gleich oder verschieden sind und Wasserstoff oder Methyl bedeuten, oder
R⁹ und R¹⁰ gemeinsam einen Cyclohexylring bilden, und
m - eine Zahl 1, 2, 3, 4 oder 5 bedeutet,
A und B Wasserstoff oder Fluor bedeuten,
sowie deren Salze.

4. Verfahren nach Anspruch 1 zur Herstellung von (+)-4-[(2-Chinolin-2-yl-methoxy)phenyl]-2-cyclopentyl-essigsäure.

5. Verfahren nach Anspruch 1 zur Herstellung von (-)-4-[(2-Chinolin-2-yl-methoxy)phenyl]-2-cyclopentyl-essigsäure.

6. Verfahren nach Anspruch 1, dadurch gekennzeichnet, daß man die Alkylierung der C-H aciden Verbindung in einem Temperaturbereich von 0°C bis +150°C durchführt.

## Claims (Claims for the following Contracting State(s): AT, BE, CH, DE, FR, GB, GR, IT, LI, NL, SE)

1. Substituted 4-(quinolin-2-yl-methoxy)phenyl-acetic acid derivatives of the formula 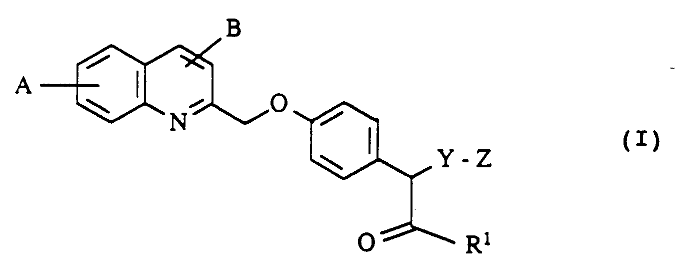 wherein
R¹ - represents a group of the formula
-OR² or 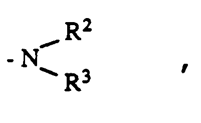 wherein
R² and R³ are identical or different and
- represent hydrogen, C₁-C₆-alkyl, benzyl, phenyl or a group of the formula 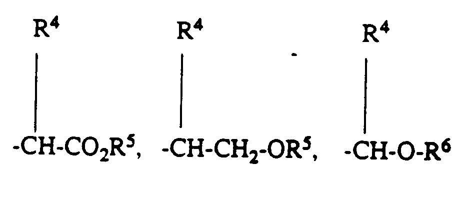 or 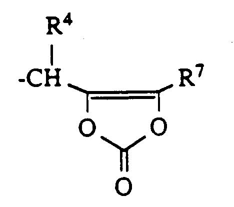 wherein
R⁴ - represents hydrogen, C₁-C₆-alkyl, phenyl or benzyl, which can optionally be substituted by hydroxyl, C₁-C₆-alkoxycarbonyl, carboxyl, C₁-C₆-alkylthio, thienyl, furyl, pyridyl, pyrimidyl, pyrazinyl, pyridazinyl, quinolyl, isoquinolyl, quinazolyl, quinoxalyl, thiazolyl, benzothiazolyl, isothiazolyl, oxazolyl, benzoxazolyl, isoxazolyl, imidazolyl, benzimidazolyl, pyrazolyl, indolyl and isoindolyl or carbamoyl,
R⁵ - represents hydrogen, C₁-C₆-alkyl, phenyl or benzyl,
R⁶ - represents a group of the formula -COR⁵ or -CO₂R⁵, wherein R⁵ has the abovementioned meaning,
and
R⁷ - represents hydrogen, C₁-C₆-alkyl or phenyl
Y - represents a group of the formula 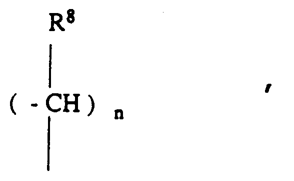 wherein
R⁸ - represents hydrogen, C₁-C₆-alkyl or phenyl
and
n - denotes a number from 0 to 5,
Z - represents norbornyl, or
represents a group of the formula 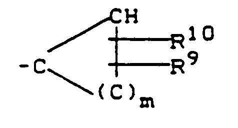 or 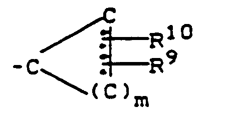 wherein
R⁹ and R¹⁰ are identical or different and denote hydrogen, C₁-C₆-alkyl or phenyl, or
R⁹ and R¹⁰ can together form a saturated carbocyclic ring having up to 6 carbon atoms and
m - denotes a number from 1 to 6,
and
A and B are identical or different and denote hydrogen, methyl, ethyl, fluorine, chlorine or bromine,
and salts thereof

2. Substituted 4-(quinolin-2-yl-methoxy)phenyl-acetic acid derivatives of the formula (I) according to Claim 1, wherein
R¹ - represents a group of the formula
-OR² or 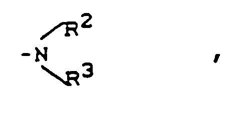 wherein
R² and R³ are identical or different and
- represent hydrogen, methyl, ethyl, propyl, isopropyl, butyl, tert.-butyl, phenyl or benzyl, or represent a group of the formula 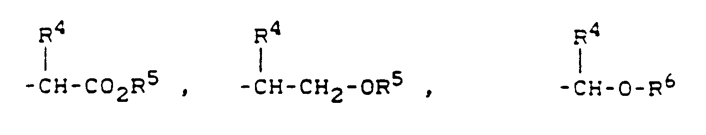 or 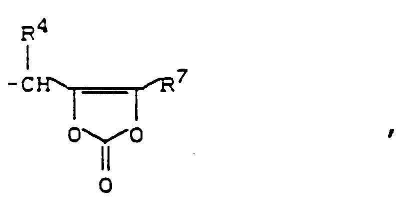 wherein
R⁴ - represents hydrogen, methyl, ethyl, propyl, isopropyl, butyl, tert.-butyl, benzyl or phenyl, which can optionally be substituted by hydroxyl, methoxycarbonyl, ethoxycarbonyl propoxycarbonyl, carboxyl, methylthio, ethylthio, propylthio, imidazolyl or carbamoyl,
R⁵ - represents hydrogen, methyl, ethyl, propyl, isopropyl, butyl, tert.-butyl, phenyl or benzyl,
R⁶ - represents a group of the formula -COR⁵ or -CO₂R⁵, wherein R⁵ has the abovementioned meaning,
and
R⁷ - represents hydrogen, methyl, ethyl, propyl, isopropyl, butyl, tert.-butyl or phenyl,
Y - represents a group of the formula 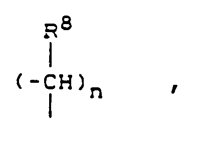 wherein
R⁸ - represents hydrogen, methyl, ethyl, propyl, isopropyl, butyl, tert.-butyl or phenyl,
and
n - denotes a number from 0 to 5,
Z - represents norbornyl, or
represents a group of the formula 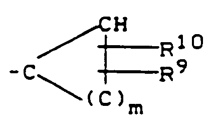 or 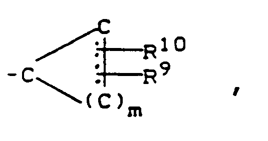 wherein
R⁹ and R¹⁰ are identical or different and denote hydrogen, methyl, ethyl, n-propyl, iso-propyl, butyl or tert.-butyl, or
R⁹ and R¹⁰ can together form a saturated carbocyclic ring having up to 6 carbon atoms and
m - denotes a number from 1 to 6,
and
A and B are identical or different and denote hydrogen, methyl, ethyl, fluorine or chlorine,
and salts thereof.

3. Substituted 4-(quinolin-2-yl-methoxy)phenylacetic acid derivatives according to Claim 1, wherein
R¹ - represents a group of the formula
-OR² or 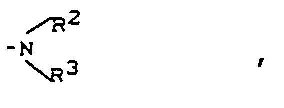 wherein
R² and R³ are identical or different and
- represent hydrogen or methyl, or represent a group of the formula 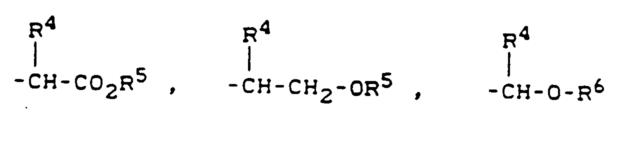 or 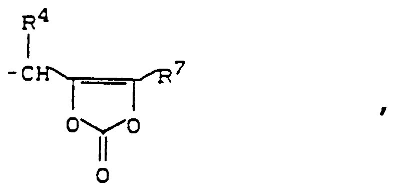 wherein
R⁴ - represents hydrogen, methyl or phenyl,
R⁵ - represents hydrogen, methyl, ethyl, tert.-butyl or benzyl,
R⁶ - represents a group of the formula -COR⁵, wherein R⁵ has the abovementioned meaning, and
R⁷ - represents methyl,
Y - represents a group of the formula 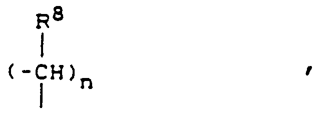 wherein
R⁸ - represents hydrogen or methyl,
and
n - denotes a number 0 or 1,
Z - represents norbornyl or
represents a group of the formula 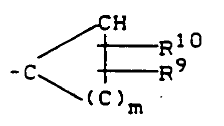 or 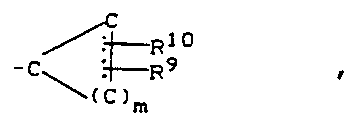 wherein
R⁹ and R¹⁰ are identical or different and denote hydrogen or methyl, or
R⁹ and R¹⁰ together form a cyclohexyl ring, and
m - denotes a number 1, 2, 3, 4 or 5,
and
A and B denote hydrogen or fluorine,
and salts thereof.

4. (+)-4-[(2-Quinolin-2-yl-methoxy)phenyl]-2-cyclopentyl-acetic acid according to Claim 1.

5. (-)-4-[(2-Quinolin-2-yl-methoxy)phenyl]-2-cyclopentyl-acetic acid according to Claim 1.

6. Process for the preparation of substituted 4-(quinolin-2-yl-methoxy)phenyl-acetic acid derivatives according to Claim 1, characterized in that
[A] 4-(quinolin-2-yl-methoxy)phenylacetic acid esters of the general formula (Ia) 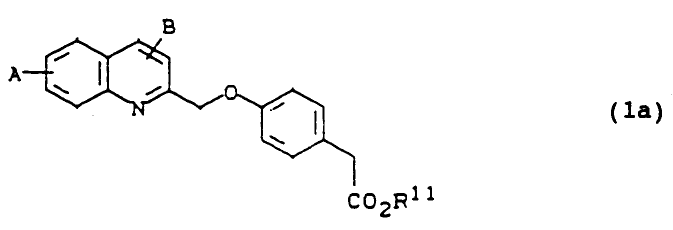 in which
R¹¹ - represents alkyl and
A and B have the abovementioned meaning,
are alkylated with compounds of the general formula (II)
Y-Z-X (II)
in which
Y and Z have the abovementioned meaning and
X - represents chlorine, bromine or iodine,
and in the case of the acids the esters are hydrolyzed,
or in that
[B] the acids of the general formula (Ib) 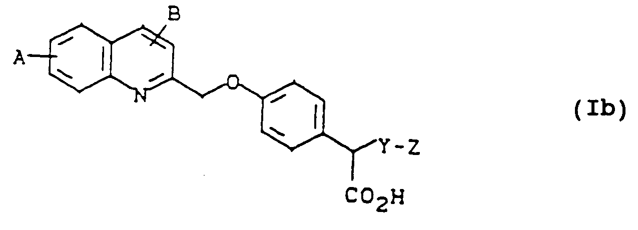 in which
A, B, Y and Z have the abovementioned meaning,
are esterified with compounds of the general formula (III)
X-R¹² (III)
in which
R¹² - represents a group of the formula 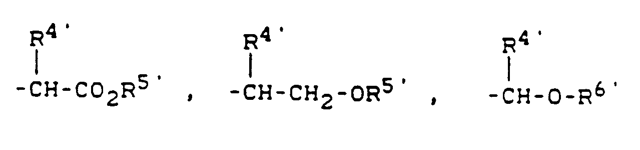 or 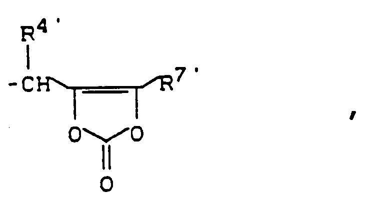 wherein
R⁴' - represents alkyl, aralkyl or aryl, which can optionally be substituted by hydroxyl, carboxyl, alkoxycarbonyl, alkylthio, heteroaryl or aminocarbonyl,
R⁵' - represents alkyl, aryl or aralkyl,
R⁶' - represents a group of the formula -COR⁵' or -CO₂R⁵', wherein
R⁵' has the abovementioned meaning,
R⁷' - represents alkyl or aryl,
and
X has the abovementioned meaning,
and in the case of the acids the esters are subjected to hydrogenolytic cleavage,
or in that
[C] the acids of the general formula (Ib) are amidated with amines of the general formula (IV) 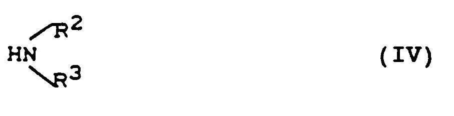 in which
R² and R³ have the abovementioned meaning,
with the proviso that R⁵ does not denote hydrogen if R² or R³ represents the group 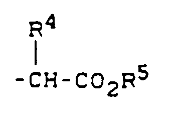 wherein
R⁴ and R⁵ have the abovementioned meaning,
in the presence of customary activating reagents, and in the case of the acids the esters are hydrolyzed,
or in that
[D] phenols of the general formula (V) 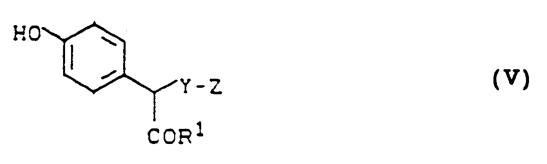 in which
R¹, Y and Z have the abovementioned meaning,
are etherified with
2-halogenomethylquinolines of the formula (VI) 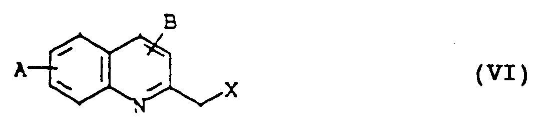 in which
A, B and X have the abovementioned meaning,
and in the case of the acids the esters are hydrolyzed.

7. Process according to Claim 6, characterized in that it is carried out in the temperature range from 0 to 150°C.

8. Medicaments containing substituted 4-(quinolin-2-yl-methoxy)phenyl-acetic acid derivatives according to Claims 1 to 5.

9. Medicaments according to Claim 8, containing 0.5 to 90% by weight of substituted 4-(quinolin-2-yl-methoxy)phenyl-acetic acid derivatives.

10. Use of substituted 4-(quinolin-2-yl-methoxy)phenyl-acetic acid derivatives according to Claims 1 to 5 for the preparation of medicaments.

## Claims (Claims for the following Contracting State(s): ES)

1. Process for the preparation of substituted 4-(quinolin-2-yl-methoxy)phenyl-acetic acid derivatives of the formula 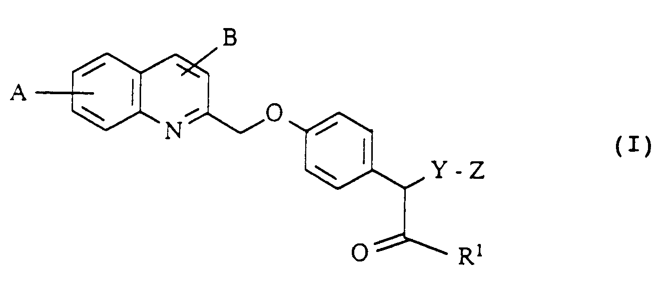 wherein
R¹ - represents a group of the formula
-OR² or 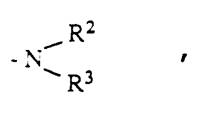 wherein
R² and R³ are identical or different and
- represent hydrogen, C₁-C₆-alkyl, benzyl, phenyl or a group of the formula 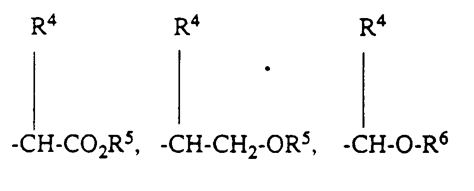 or 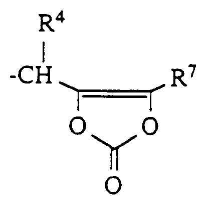 wherein
R⁴ - represents hydrogen, C₁-C₆-alkyl, phenyl or benzyl, which can optionally be substituted by hydroxyl, C₁-C₆-alkoxycarbonyl, carboxyl, C₁-C₆-alkylthio, thienyl, furyl, pyridyl, pyrimidyl, pyrazinyl, pyridazinyl, quinolyl, isoquinolyl, quinazolyl, quinoxalyl, thiazolyl, benzothiazolyl, isothiazolyl, oxazolyl, benzoxazolyl, isoxazolyl, imidazolyl, benzimidazolyl, pyrazolyl, indolyl and isoindolyl or carbamoyl,
R⁵ - represents hydrogen, C₁-C₆-alkyl, phenyl or benzyl,
R⁶ - represents a group of the formula -COR⁵ or -CO₂R⁵, wherein R⁵ has the abovementioned meaning,
and
R⁷ - represents hydrogen, C₁-C₆-alkyl or phenyl
Y - represents a group of the formula 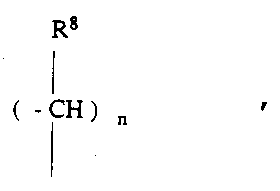 wherein
R⁸ - represents hydrogen, C₁-C₆-alkyl or phenyl
and
n - denotes a number from 0 to 5,
Z - represents norbornyl, or
represents a group of the formula 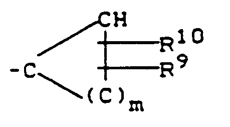 or 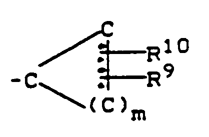 wherein
R⁹ and R¹⁰ are identical or different and denote hydrogen, C₁-C₆-alkyl or phenyl, or
R⁹ and R¹⁰ can together form a saturated carbocyclic ring having up to 6 carbon atoms and
m - denotes a number from 1 to 6,
and
A and B are identical or different and denote hydrogen, methyl, ethyl, fluorine, chlorine or bromine,
and salts thereof, characterized in that,
[A] 4-(quinolin-2-yl-methoxy)phenylacetic acid esters of the general formula (Ia) 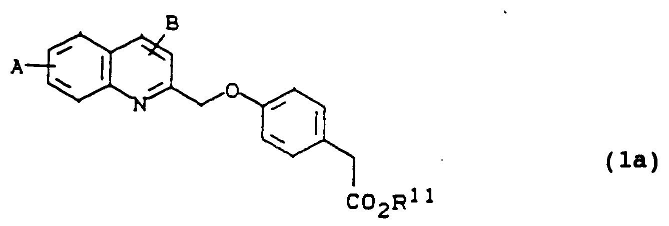 in which
R¹¹ - represents alkyl and
A and B have the abovementioned meaning,
are alkylated with compounds of the general formula (II)
Y-Z-X (II)
in which
Y and Z have the abovementioned meaning and
X - represents chlorine, bromine or iodine,
and in the case of the acids the esters are hydrolyzed,
or in that
[B] the acids of the general formula (Ib) 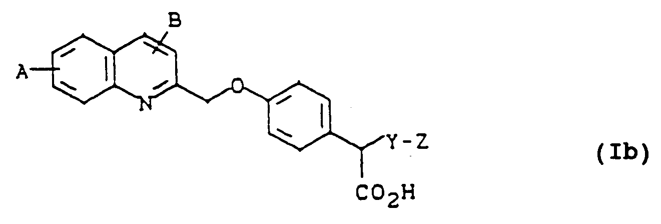 in which
A, B, Y and Z have the abovementioned meaning,
are esterified with compounds of the general formula (III)
X-R¹² (III)
in which
R¹² - represents a group of the formula 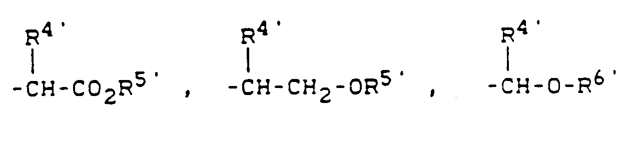 or 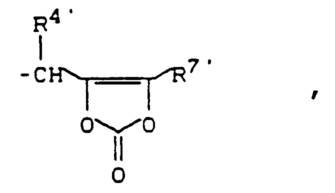 wherein
R⁴' - represents alkyl, aralkyl or aryl, which can optionally be substituted by hydroxyl, carboxyl, alkoxycarbonyl, alkylthio, hetero aryl or
R⁵' - represents alkyl, aryl or aralkyl,
R⁶' - represents a group of the formula -COR⁵' or -CO₂R⁵', wherein
R⁵' has the abovementioned meaning,
R⁷' - represents alkyl or aryl,
and
X has the abovementioned meaning,
and in the case of the acids the esters are subjected to hydrogenolytic cleavage,
or in that
[C] the acids of the general formula (Ib) are amidated with amines of the general formula (IV) 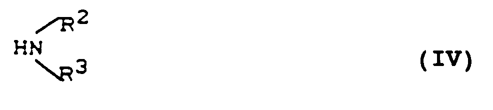 in which
R² and R³ have the abovementioned meaning,
with the proviso that R⁵ does not denote hydrogen if R² or R³ represents the group 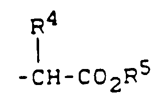 wherein
R⁴ and R⁵ have the abovementioned meaning,
in the presence of customary activating reagents, and in the case of the acids the esters are hydrolyzed,
or in that
[D] phenols of the general formula (V) 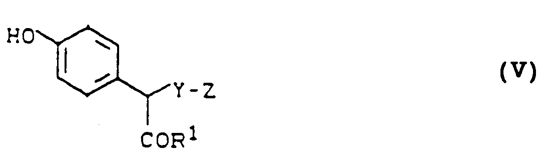 in which
R¹, Y and Z have the abovementioned meaning,
are etherified with
2-halogenomethylquinolines of the formula (VI) 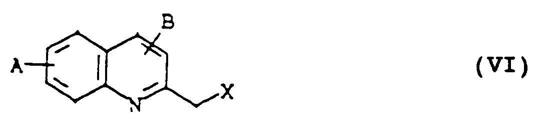 in which
A, B and X have the abovementioned meaning,
and in the case of the acids the esters are hydrolyzed.

2. Process according to Claim 1 for the preparation of substituted 4-(quinolin-2-yl-methoxy)phenylacetic acid derivatives of the formula (I) according to Claim 1, wherein
R¹ - represents a group of the formula
-OR² or 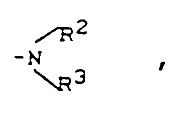 wherein
R² and R³ are identical or different and
- represent hydrogen, methyl, ethyl, propyl, isopropyl, butyl, tert.-butyl, phenyl or benzyl, or represent a group of the formula 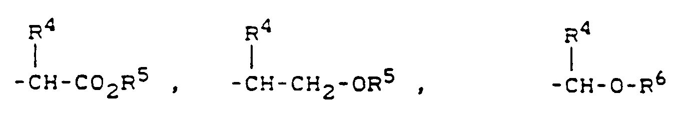 or 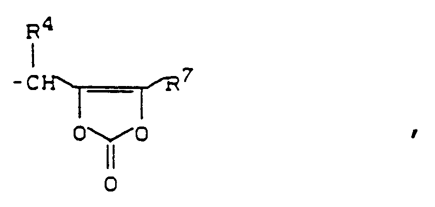 wherein
R⁴ - represents hydrogen, methyl, ethyl, propyl, isopropyl, butyl, tert.-butyl, benzyl or phenyl, which can optionally be substituted by hydroxyl, methoxycarbonyl, ethoxycarbonyl propoxycarbonyl, carboxyl, methylthio, ethylthio, propylthio, imidazolyl or carbomoyl,
R⁵ - represents hydrogen, methyl, ethyl, propyl, isopropyl, butyl, tert.-butyl, phenyl or benzyl,
R⁶ - represents a group of the formula -COR⁵ or -CO₂R⁵, wherein R⁵ has the abovementioned meaning,
and
R⁷ - represents hydrogen, methyl, ethyl, propyl, isopropyl, butyl, tert.-butyl or phenyl,
Y - represents a group of the formula 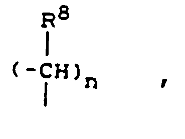 wherein
R⁸ - represents hydrogen, methyl, ethyl, propyl, isopropyl, butyl, tert.-butyl or phenyl,
and
n - denotes a number from 0 to 5,
Z - represents norbornyl, or
represents a group of the formula 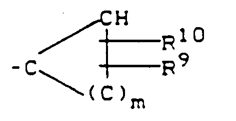 or 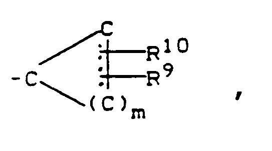 wherein
R⁹ and R¹⁰ are identical or different and denote hydrogen, methyl, ethyl, n-propyl, iso-propyl, butyl or tert.-butyl, or
R⁹ and R¹⁰ can together form a saturated carbocyclic ring having up to 6 carbon atoms and
m - denotes a number from 1 to 6,
and
A and B are identical or different and denote hydrogen, methyl, ethyl, fluorine or chlorine,
and salts thereof.

3. Process according to Claim 1 for the preparation of substituted 4-(quinolin-2-yl-methoxy)phenyl-acetic acid derivatives of the formula (I) according to Claim 1, wherein
R¹ - represents a group of the formula
-OR² or 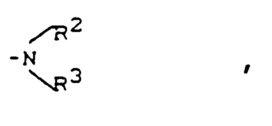 wherein
R² and R³ are identical or different and
- represent hydrogen or methyl, or represent a group of the formula 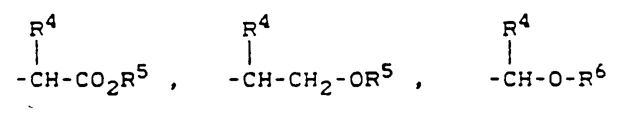 or 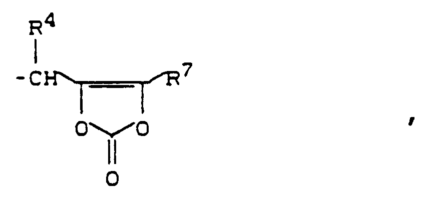 wherein
R⁴ - represents hydrogen, methyl or phenyl,
R⁵ - represents hydrogen, methyl, ethyl, tert.-butyl or benzyl,
R⁶ - represents a group of the formula -COR⁵, wherein R⁵ has the abovementioned meaning, and
R⁷ - represents methyl,
Y - represents a group of the formula 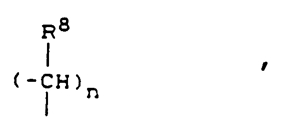 wherein
R⁸ - represents hydrogen or methyl,
and
n - denotes a number 0 or 1,
Z - represents norbornyl or
represents a group of the formula 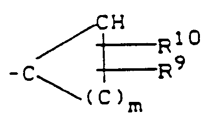 or 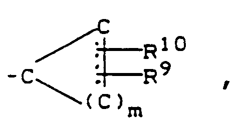 wherein
R⁹ and R¹⁰ are identical or different and denote hydrogen or methyl, or
R⁹ and R¹⁰ together form a cyclohexyl ring, and
m - denotes a number 1, 2, 3, 4 or 5,
and
A and B denote hydrogen or fluorine,
and salts thereof.

4. Process according to Claim 1 for the preparation of (+)-4-[(2-quinolin-2-yl-methoxy)phenyl]-2-cyclo-pentyl-acetic acid.

5. Process according to Claim 1 for the preparation of (-)-4-[(2-quinolin-2-yl-methoxy)phenyl]-2-cyclo-pentyl-acetic acid.

6. Process according to Claim 1 characterized in that the alkylation of the C-H acid compound is carried out in a temperature range from 0°C to +150°C.

## Revendications (Revendications pour l'(les) Etat(s) contractant(s) suivant(s): AT, BE, CH, DE, FR, GB, GR, IT, LI, NL, SE)

1. Dérivés substitués d'acide 4-(quinoléine-2-yl-méthoxy)phényl-acétique de formule 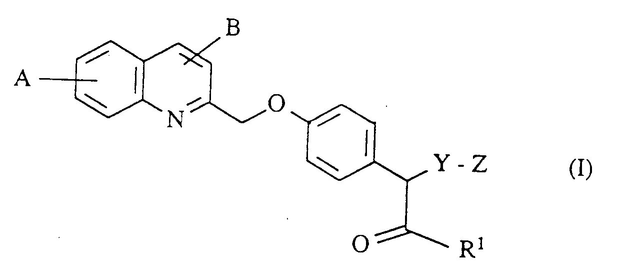 dans laquelle
R¹ - est un groupe de formule
-OR² ou 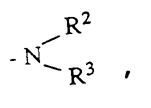 dans laquelle
R² et R³ sont identiques ou différents et représentent
- l'hydrogène, un groupe alkyle en C₁ à C₆, benzyle, phényle ou un groupe de formule 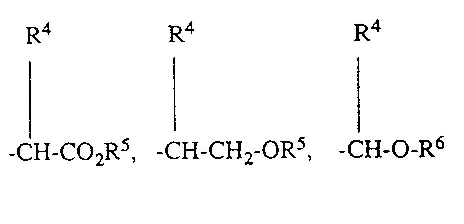 ou 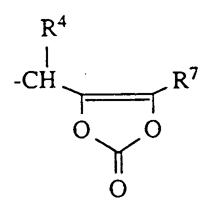 dans laquelle
R⁴ - est l'hydrogène, un groupe alkyle en C₁ à C₆, phényle ou benzyle, qui peut porter le cas échéant un substituant hydroxy, (alkoxy en C₁ à C₆)carbonyle, carboxyle, alkylthio en C₁ à C₆, thiényle, furyle, pyridyle, pyrimidyle, pyrazinyle, pyridazinyle, quinoléyle, isoquinoléyle, quinazolyle, quinoxalyle, thiazolyle, benzothiazolyle, isothiazolyle, oxazolyle, benzoxazolyle, isoxazolyle, imidazolyle, benzimidazolyle, pyrazolyle, indolyle et isoindolyle ou carbamoyle,
R⁵ - représente l'hydrogène, un groupe alkyle en C₁ à C₆, phényle ou benzyle,
R⁶ - est un groupe de formule -COR⁵ ou -CO₂R⁵ dans laquelle R⁵ a la définition indiquée ci-dessus,
et
R⁷ - est l'hydrogène, un groupe alkyle en C₁ à C₆ ou phényle,
Y - est un groupe de formule 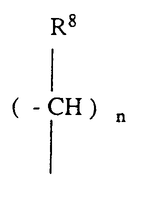 dans laquelle
R⁸ - représente l'hydrogène, un groupe alkyle en C₁ à C₆ ou phényle,
et
n - est un nombre de 0 à 5,
Z - est un groupe norbornyle ou un groupe de formule 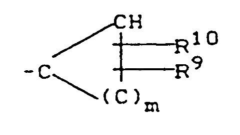 ou 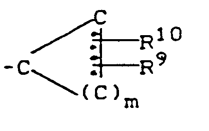 dans laquelle
R⁹ et R¹⁰ sont identiques ou différents et représentent l'hydrogène, un groupe alkyle en C₁ à C₆ ou phényle, ou bien
R⁹ et R¹⁰ peuvent former conjointement un noyau carbocyclique saturé ayant jusqu'à 6 atomes de carbone et
m est un nombre de 1 à 6,
et
A et B sont identiques ou différents et représentent l'hydrogène, un groupe méthyle, éthyle, le fluor, le chlore ou le brome,
ainsi que leurs sels.

2. Dérivés substitués d'acide 4-(quinoléine-2-yl-méthoxy)phényl-acétique de formule (I) suivant la revendication 1, dans lesquels
R¹ - est un groupe de formule
-OR² ou 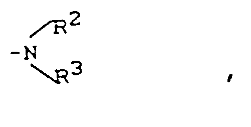 où
R² et R³ sont identiques ou différents et représentent
- l'hydrogène, un groupe méthyle, éthyle, propyle, isopropyle, butyle, tertio-butyle, phényle ou benzyle, ou un groupe de formule 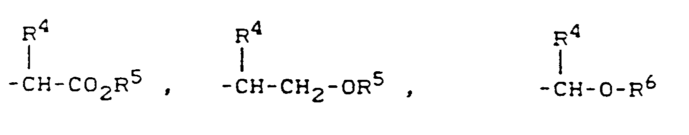 ou 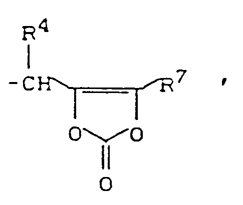 où
R⁴ - représente l'hydrogène, un groupe méthyle, éthyle, propyle, isopropyle, butyle, tertio-butyle, benzyle ou phényle qui peut porter le cas échéant un substituant hydroxy, méthoxycarbonyle, éthoxycarbonyle, propoxycarbonyle, carboxyle, méthylthio, éthylthio, propylthio, imidazolyle ou carbamoyle
R⁵ - représente l'hydrogène, un groupe méthyle, éthyle, propyle, iso-propyle, butyle, tertio-butyle, phényle ou benzyle,
et
R⁶ - est un groupe de formule -COR⁵ ou -CO₂R⁵, dans laquelle R⁵ a la définition indiquée ci-dessus
et
R⁷ - représente l'hydrogène, un groupe méthyle, éthyle, propyle, iso-propyle, butyle, tertio-butyle ou phényle,
Y - est un groupe de formule 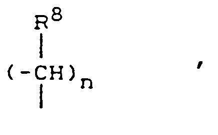 dans laquelle
R⁸ - est l'hydrogène, un groupe méthyle, éthyle, propyle, isopropyle, butyle, tertio-butyle ou phényle,
et
n - est un nombre de 0 à 5,
Z - est le groupe norbornyle ou un groupe de formule 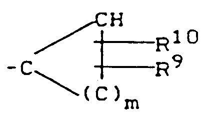 ou 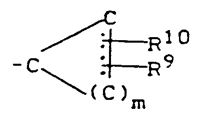 dans laquelle
R⁹ et R¹⁰ sont identiques ou différents et représentent l'hydrogène, un groupe méthyle, éthyle, n-propyle, isopropyle, butyle ou tertio-butyle, ou bien
R⁹ et R¹⁰ peuvent former conjointement un noyau carbocyclique saturé ayant jusqu'à 6 atomes de carbone et
m - est un nombre de 1 à 6,
et
A et B sont identiques ou différents et représentent l'hydrogène, un groupe méthyle, éthyle, le fluor ou le chlore,
ainsi que leurs sels.

3. Dérivés substitués d'acide 4-(quinoléine-2-yl-méthoxy)phényl-acétique suivant la revendication 1, dans lesquels
R¹ - est un groupe de formule
-OR² ou 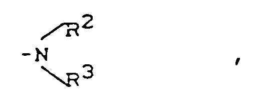 où
R² et R³ sont identiques ou différents et représentent
- l'hydrogène ou le groupe méthyle, ou bien
- un groupe de formule 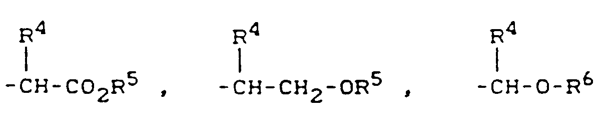 ou 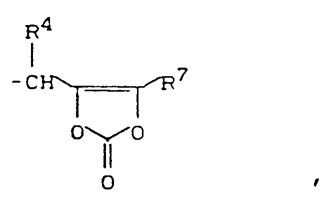 dans laquelle
R⁴ - est l'hydrogène, un groupe méthyle ou phényle,
R⁵ - est l'hydrogène, un groupe méthyle, éthyle, tertio-butyle ou benzyle,
R⁶ - est un groupe de formule -COR⁵, R⁵ ayant la définition indiquée ci-dessus, et
R⁷ - est un groupe méthyle,
Y - est un groupe de formule 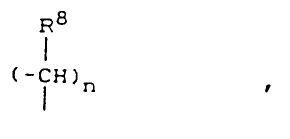 dans laquelle
R⁸ - est l'hydrogène ou le groupe méthyle,
et
n - est le nombre 0 ou 1,
Z - est un groupe norbornyle, ou
un groupe de formule 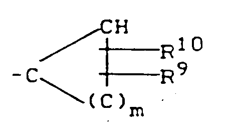 ou 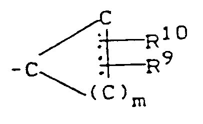 dans laquelle
R⁹ et R¹⁰ sont identiques ou différents et représentent l'hydrogène ou le groupe méthyle, ou bien
R⁹ et R¹⁰ forment conjointement un noyau cyclohexyle,
et
m - est le nombre 1, 2, 3, 4 ou 5,
A et B représentent l'hydrogène ou le fluor,
ainsi que leurs sels.

4. L'acide (+)-4-[(2-quinoléine-2-yl-méthoxy)-phényl]-2-cyclopentyl-acétique suivant la revendication 1.

5. L'acide (-)-4-[(2-quinoléine-2-yl-méthoxy)-phényl]-2-cyclopentyl-acétique suivant la revendication 1.

6. Procédé de production de dérivés substitués d'acide 4-(quinoléine-2-yl-méthoxy)phényl-acétique suivant la revendication 1, caractérisé en ce que
[A] On alkyle un ester d'acide 4-(quinoléine-2-yl-méthoxy)phényl-acétique de formule générale (Ia) 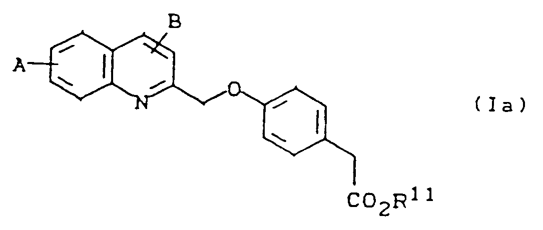 dans laquelle
R¹¹ - est un groupe alkyle et
A et B ont la définition indiquée ci-dessus,
avec des composés de formule générale (II)
Y-Z-X (II)
dans laquelle
Y et Z ont la définition indiquée ci-dessus et
X - représente le chlore, le brome ou l'iode,
et dans le cas des acides, on saponifie les esters, ou bien
[B] on estérifie les acides de formule générale (Ib) 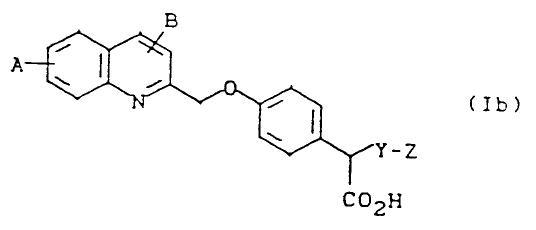 dans laquelle
A, B, Y et Z ont la définition indiquée ci-dessus,
avec des composés de formule générale (III)
X-R¹² (III)
dans laquelle
R¹² - est un groupe de formule 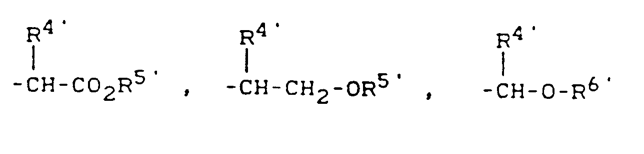 ou 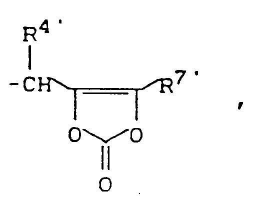 où
R⁴' - représente un groupe alkyle, aralkyle ou aryle qui peut être substitué le cas échéant par un radical hydroxy, carboxyle, alkoxycarbonyle, alkylthio, hétéroaryle ou amino-carbonyle,
R⁵' - est un groupe alkyle, aryle ou aralkyle,
R⁶' est un groupe de formule -COR⁵' ou -CO₂R⁵', dans laquelle R⁵' a la définition indiquée ci-dessus,
R⁷' - est un groupe alkyle ou aryle,
et
X a la définition indiquée ci-dessus,
et dans le cas des acides, on soumet les esters à une scission par hydrogénolyse,
ou bien
[C] on effectue l'amidation, en présence de réactifs classiques d'activation, des acides de formule générale (Ib) avec des amines de formule générale (IV) 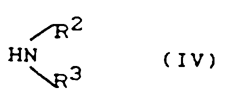 dans laquelle
R² et R³ ont la définition indiquée ci-dessus, sous réserve que R⁵ ne représente pas l'hydrogène lorsque R² ou R³ représente le groupe 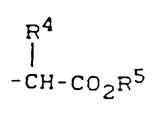 R⁴ et R⁵ ayant la définition indiquée ci-dessus,
et dans le cas des acides, on saponifie les esters,
ou bien
[D] On éthérifie les phénols de formule générale (V) 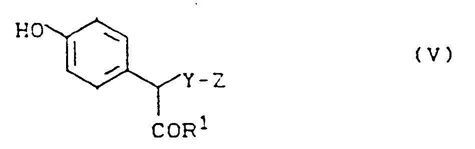 dans laquelle
R¹, Y et Z ont la définition indiquée ci-dessus avec
des 2-halogénométhylquinoléines de formule (VI) 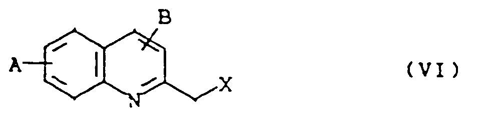 dans laquelle
A, B et X ont la définition indiquée ci-dessus,
et dans le cas des acides, on saponifie les éthers.

7. Procédé suivant la revendication 6, caractérisé en ce qu'il est mis en oeuvre dans la plage de températures de 0 à 150°C.

8. Médicament contenant des dérivés substitués d'acide 4-(quinoléine-2-yl-méthoxy)phényl-acétique suivant les revendications 1 à 5.

9. Médicament suivant la revendication 8, contenant 0,5 à 90 % en poids de dérivés substitués d'acide 4-(quinoléine-2-yl-méthoxy)phényl-acétique.

10. Utilisation de dérivés substitués d'acide 4-(quinoléine-2-yl-méthoxy)phényl-acétique suivant les revendications 1 à 5 pour la préparation de médicaments.

## Revendications (Revendications pour l'(les) Etat(s) contractant(s) suivant(s): ES)

1. Procédé de production de dérivés substitués d'acide 4-(quinoléine-2-yl-méthoxy)phényl-acétique de formule 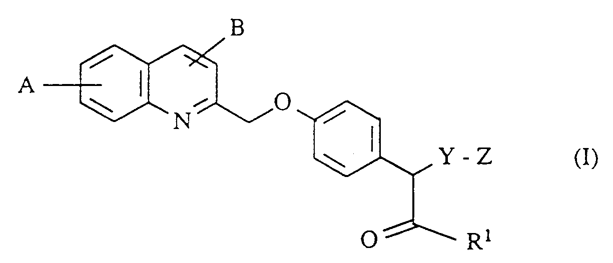 dans laquelle
R¹ - est un groupe de formule
-OR² ou 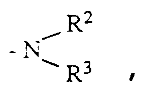 dans laquelle
R² et R³ sont identiques ou différents et représentent
- l'hydrogène, un groupe alkyle en C₁ à C₆, benzyle, phényle ou un groupe de formule 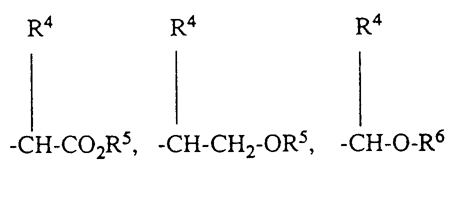 ou 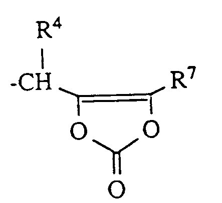 dans laquelle
R⁴ - est l'hydrogène, un groupe alkyle en C₁ à C₆, phényle ou benzyle, qui peut porter le cas échéant un substituant hydroxy, (alkoxy en C₁ à C₆)carbonyle, carboxyle, alkylthio en C₁ à C₆, thiényle furyle, pyridyle, pyrimidyle, pyrazinyle, pyridazinyle, quinoléyle, isoquinoléyle, quinazolyle, quinoxalyle, thiazolyle, benzothiazolyle, isothiazolyle, oxazolyle, benzoxazolyle, isoxazolyle, imidazolyle, benzimidazolyle, pyrazolyle, indolyle et isoindolyle ou carbamoyle,
R⁵ - représente l'hydrogène, un groupe alkyle en C₁ à C₆, phényle ou benzyle,
R⁶ - est un groupe de formule -COR⁵ ou -CO₂R⁵ dans laquelle R⁵ a la définition indiquée ci-dessus,
et
R⁷ - est l'hydrogène, un groupe alkyle en C₁ à C₆ ou phényle,
Y - est un groupe de formule 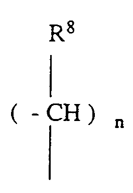 dans laquelle
R⁸ - représente l'hydrogène, un groupe alkyle en C₁ à C₆ ou phényle,
et
n - est un nombre de 0 à 5,
Z - est un groupe norbornyle ou un groupe de formule 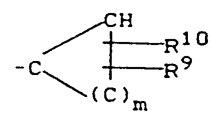 ou 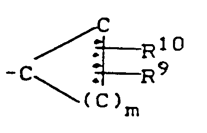 dans laquelle
R⁹ et R¹⁰ sont identiques ou différents et représentent l'hydrogène, un groupe alkyle en C₁ à C₆ ou phényle, ou bien
R⁹ et R¹⁰ peuvent former conjointement un noyau carbocyclique saturé ayant jusqu'à 6 atomes de carbone et
m est un nombre de 1 à 6,
et
A et B sont identiques ou différents et représentent l'hydrogène, un groupe méthyle, éthyle, le fluor, le chlore ou le brome,
ainsi que leurs sels, caractérisé en ce que
[A] On alkyle un ester d'acide 4-(quinoléine-2-yl-méthoxy)phényl-acétique de formule générale (Ia) 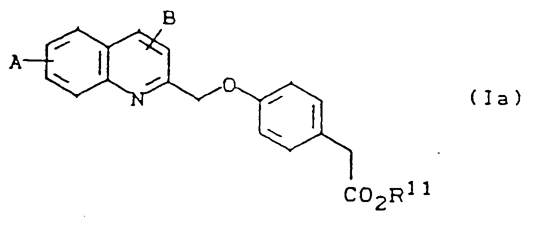 dans laquelle
R¹¹ - est un groupe alkyle et
A et B ont la définition indiquée ci-dessus,
avec des composés de formule générale (II)
Y-Z-X (II)
dans laquelle
Y et Z ont la définition indiquée ci-dessus et
X - représente le chlore, le brome ou l'iode,
et dans le cas des acides, on saponifie les esters, ou bien
[B] on estérifie les acides de formule générale (Ib) 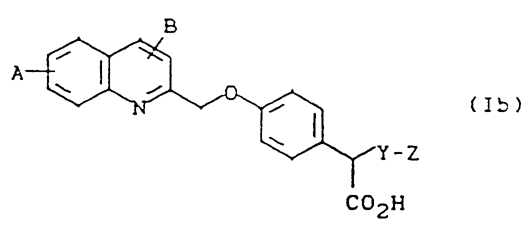 dans laquelle
A, B, Y et Z ont la définition indiquée ci-dessus,
avec des composés de formule générale (III)
X-R¹² (III)
dans laquelle
R¹² - est un groupe de formule 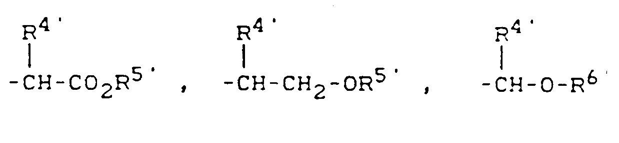 ou 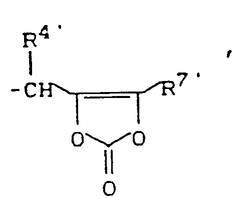 où
R⁴' - représente un groupe alkyle, aralkyle ou aryle qui peut être substitué le cas échéant par un radical hydroxy, carboxyle, alkoxycarbonyle, alkylthio, hétéroaryle ou amino-carbonyle,
R⁵' - est un groupe alkyle, aryle ou aralkyle,
R⁶' - est un groupe de formule -COR⁵' ou -CO₂R⁵', dans laquelle
R⁵' a la définition indiquée ci-dessus,
R⁷' - est un groupe alkyle ou aryle,
et
X a la définition indiquée ci-dessus,
et dans le cas des acides, on soumet les esters à une scission par hydrogénolyse, ou bien
[C] on effectue l'amidation, en présence de réactifs classiques d'activation, des acides de formule générale (Ib) avec des amines de formule générale (IV) 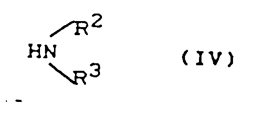 dans laquelle
R² et R³ ont la définition indiquée ci-dessus,
sous réserve que R⁵ ne représente pas l'hydrogène lorsque R² ou R³ représente le groupe 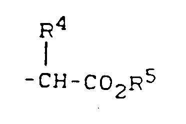 R⁴ et R⁵ ayant la définition indiquée ci-dessus,
et dans le cas des acides, on saponifie les esters,
ou bien
[D] On éthérifie les phénols de formule générale (V) 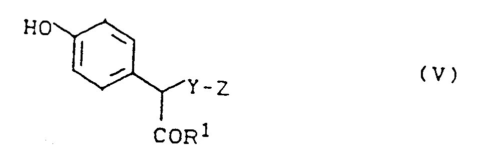 dans laquelle
R¹, Y et Z ont la définition indiquée ci-dessus avec
des 2-halogénométhylquinoléines de formule (VI) 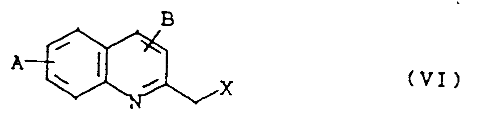 dans laquelle
A, B et X ont la définition indiquée ci-dessus,
et dans le cas des acides, on saponifie les éthers.

2. Procédé suivant la revendication 1 pour la préparation de dérivés substitués d'acide 4-(quinoléine-2-yl-méthoxy)phényl-acétique de formule suivant la revendication 1, où
R¹ - est un groupe de formule
-OR² ou 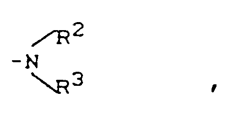 où
R² et R³ sont identiques ou différents et représentent
- l'hydrogène, un groupe méthyle, éthyle, propyle, isopropyle, butyle, tertio-butyle, phényle ou benzyle, ou un groupe de formule 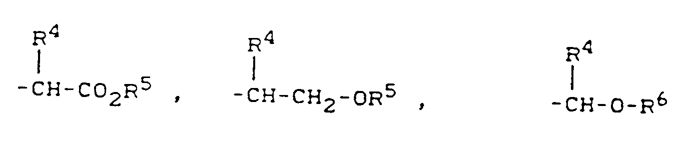 ou 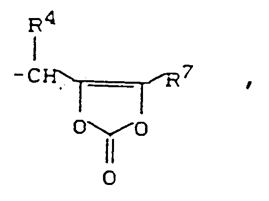 où
R⁴ - représente l'hydrogène, un groupe méthyle, éthyle, propyle, isopropyle, butyle, tertio-butyle, benzyle ou phényle qui peut porter le cas échéant un substituant hydroxy, méthoxycarbonyle, éthoxycarbonyle, propoxycarbonyle, carboxyle, méthylthio, éthylthio, propylthio, imidazolyle ou carbamoyle
R⁵ - représente l'hydrogène, un groupe méthyle, éthyle, propyle, iso-propyle, butyle, tertio-butyle, phényle ou benzyle,
et
R⁶ - est un groupe de formule -COR⁵ ou -CO₂R⁵, dans laquelle R⁵ a la définition indiquée ci-dessus
et
R⁷ - représente l'hydrogène, un groupe méthyle, éthyle, propyle, iso-propyle, butyle, tertio-butyle ou phényle,
Y - est un groupe de formule 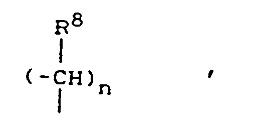 dans laquelle
R⁸ - est l'hydrogène, un groupe méthyle, éthyle, propyle, isopropyle, butyle, tertio-butyle ou phényle,
et
n - est un nombre de 0 à 5,
Z - est le groupe norbornyle ou un groupe de formule 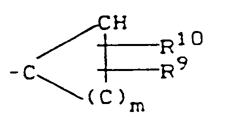 ou 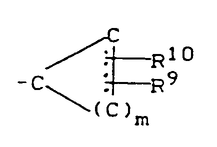 dans laquelle
R⁹ et R¹⁰ sont identiques ou différents et représentent l'hydrogène, un groupe méthyle, éthyle, n-propyle, isopropyle, butyle ou tertio-butyle, ou bien
R⁹ et R¹⁰ peuvent former conjointement un noyau carboxyclique saturé ayant jusqu'à 6 atomes de carbone et
m - est un nombre de 1 à 6,
et
A et B sont identiques ou différents et représentent l'hydrogène, un groupe méthyle, éthyle, le fluor ou le chlore,
ainsi que leurs sels.

3. Procédé suivant la revendication 1, pour la préparation de dérivés substitués d'acide 4-(quinoléine-2-yl-méthoxy)phényl-acétique de formule suivant la revendication 1, où
R¹ - est un groupe de formule
-OR² ou 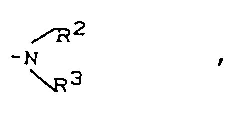 où
R² et R³ sont identiques ou différents et représentent
- l'hydrogène ou le groupe méthyle, ou bien
- un groupe de formule 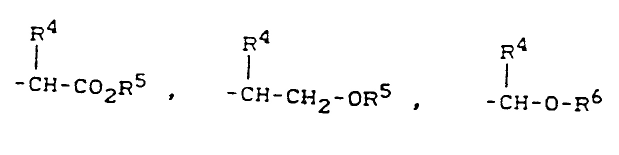 ou 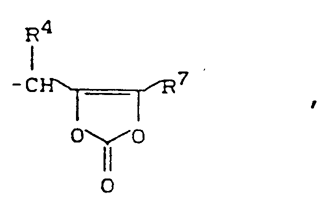 dans laquelle
R⁴ - est l'hydrogène, un groupe méthyle ou phényle,
R⁵ - est l'hydrogène, un groupe méthyle, éthyle, tertio-butyle ou benzyle,
R⁶ - est un groupe de formule -COR⁵, R⁵ ayant la définition indiquée ci-dessus, et
R⁷ - est un groupe méthyle,
Y - est un groupe de formule 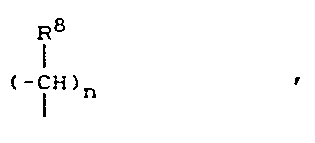 dans laquelle
R⁸ - est l'hydrogène ou le groupe méthyle,
et
n - est le nombre 0 ou 1,
Z - est un groupe norbornyle, ou
un groupe de formule 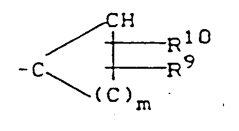 ou 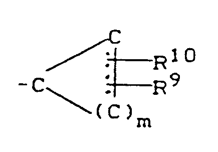 dans laquelle
R⁹ et R¹⁰ sont identiques ou différents et représentent l'hydrogène ou le groupe méthyle, ou bien
R⁹ et R¹⁰ forment conjointement un noyau cyclohexyle
et
m - est le nombre 1, 2, 3, 4 ou 5,
et
A et B représentent l'hydrogène ou le fluor,
ainsi que leurs sels.

4. Procédé suivant la revendication 1, pour l'obtention de l'acide (+)-4-[(2-quinoléine-2-yl-méthoxy)-phényl)-2-cyclopentyl-acétique.

5. Procédé suivant la revendication 1, pour l'obtention de l'acide (-)-4-[(2-quinoléine-2-yl-méthoxy)-phényl]-2-cyclopentyl-acétique.

6. Procédé suivant la revendication 1, caractérisé en ce qu'on conduit l'alkylation du composé C-H-acide dans une plage de températures de 0°C à +150°C.
